(19) Europäisches Patentamt
European Patent Office
Office européen des brevets

(11) EP 2 130 830 A1

(12) # EUROPEAN PATENT APPLICATION

(43) Date of publication:
**09.12.2009 Bulletin 2009/50**

(21) Application number: **09161744.9**

(22) Date of filing: **03.06.2009**

(51) Int Cl.:
***C07D 453/02*** (2006.01) ***A61K 31/439*** (2006.01)
***A61P 11/00*** (2006.01) ***A61P 11/06*** (2006.01)
***A61P 13/00*** (2006.01) ***A61P 1/00*** (2006.01)
***A61P 3/04*** (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **03.06.2008 IN DE13342008**

(71) Applicant: **Ranbaxy Laboratories Limited**
**Gurgaon, Haryana 122001 (IN)**

(72) Inventors:
- **Kumar, Naresh**
  **122017, Haryana (IN)**
- **Sattigeri, Jitendra A.**
  **122001, Haryana (IN)**
- **Aeron, Shelly**
  **100058, Delhi (IN)**
- **Garg, Malvika**
  **135102, Haryana (IN)**
- **Ray, Abhijit**
  **110070, Delhi (IN)**
- **Gupta, Suman**
  **122001, Haryana (IN)**
- **Malhotra, Shivani**
  **110008, Delhi (IN)**
- **Shirumalla, Raj Kumar**
  **110018, Delhi (IN)**

(74) Representative: **Cronin, Brian Harold John**
**CRONIN Intellectual Property**
**Chemin de Précossy 31**
**1260 Nyon (CH)**

(54) **Muscarinic receptor antagonists**

(57) The present invention relates generally to muscarinic receptor antagonist, which are useful, among other uses, for the treatment of various diseases of the respiratory, urinary and gastrointestinal systems mediated through muscarinic receptors. The invention also relates to the process for the preparation of disclosed compounds, pharmaceutical compositions containing the disclosed compounds and the method for treating diseases mediated through muscarinic receptors. Also provided herein are pharmaceutical composition comprising one or more muscarinic receptor antagonists and at least one other active ingredients include, but are not limited to, corticosteroids, beta agonists, leukotriene antagonists, 5-lipoxygenase inhibitors, anti-histamines, antitussives, dopamine receptor antagonists, chemokine inhibitors, p38 MAP Kinase inhibitors, and PDE-IV inhibitors.

EP 2 130 830 A1

## Description

### Field of the Invention

The present invention relates to muscarinic receptor antagonists, which are useful, among other uses, for the treatment of various diseases of the respiratory, urinary and gastrointestinal systems mediated through muscarinic receptors. The invention also relates to the processes for the preparation of the disclosed compounds, pharmaceutical compositions containing the disclosed compounds, and methods for treating diseases mediated through muscarinic receptors. Also provided herein are pharmaceutical compositions comprising one or more muscarinic receptor antagonists and at least one other active ingredients including, but not limited to, corticosteroids, beta agonists, leukotriene antagonists, 5-lipoxygenase inhibitors, antihistamines, antitussives, dopamine receptor antagonists, chemokine inhibitors, PAF, EGFR, p38 MAP kinase inhibitor and PDE-4 inhibitors.

### Background of the Invention

Muscarinic receptors belong to the superfamily of G-protein coupled receptors and five molecularly distinct subtypes are known to exist ($M_1$, $M_2$, $M_3$, $M_4$ and $M_5$). These receptors are widely distributed on multiple organs and tissues and are critical to the maintenance of central and peripheral cholinergic neurotransmission. The regional distribution of these receptor sub-types in the brain and other organs has been documented (for example, the $M_1$ subtype is located primarily in neuronal tissues such as cerebral cortex and autonomic ganglia, the $M_2$ subtype is present mainly in the heart and bladder smooth muscle, and the $M_3$ subtype is located predominantly on smooth muscle and salivary glands (Nature, 323, p.411 (1986); Science, 237, p.527 (1987)).

A review in Curr. Opin. Chem. Biol., 3, p. 426 (1999), as well as in Trends in Pharmacol. Sci., 22, p.409 (2001) by Eglen *et al.*, describes the biological potentials of modulating muscarinic receptor subtypes by ligands in different diseases conditions, such as Alzheimer's diseases, pain, urinary disease condition, chronic obstructive pulmonary diseases, and the like.

The pharmacological and medical aspects of the muscarinic class of acetylcholine agonists and antagonists are presented in a review in Molecules, 6, p.142 (2001). Birdsall et al. in Trends in Pharmacol. Sci., 22, p.215 (2001) has also summarized the recent developments on the role of different muscarinic receptor subtypes using different muscarinic receptor of knock out mice.

Almost all the smooth muscles express a mixed population of $M_2$ and $M_3$ receptors. Although the $M_2$-receptor are the predominate cholinoreceptors, the smaller population of $M_3$-receptor appears to be the most functionally important as they mediate the direct contraction of these smooth muscle. Muscarinic receptor antagonist are known to be useful for treating various medical conditions associated with improper smooth muscle function, such as overactive bladder syndrome, irritable bowel syndrome and chronic obstructive pulmonary disease. However, the therapeutic utility of antimuscarinic has been limited by poor tolerability as a result of treatment related, frequent systemic adverse events such as dry mouth, constipation, blurred vision, headache, somnolence and tachycardia. Thus, there exist needs for novel muscarinic receptor antagonist that demonstrate target organ selectivity.

WO 2004/005252 discloses azabicyclo derivatives described as muscarinic receptor antagonists. WO 2004/004629, WO 2004/052857, WO 2004/067510, WO 2004/014853, WO 2004/014363 discloses 3, 6-disubstituted azabicyclo [3.1.0] hexane derivatives described as useful muscarinic receptor antagonists. WO 2004/056811 discloses flaxavate derivatives as muscarinic receptor antagonists. WO 2004/056810 discloses xanthene derivatives as muscarinic receptor antagonists. WO 2004/056767 discloses 1-substituted-3-pyrrolidine derivatives as muscarinic receptor antagonists. WO 99/14200, WO 03/027060, U.S. Patent No. 6,200,991, WO 00/56718 discloses heterocycle derivatives as muscarinic receptor antagonists. WO 2004/089363, WO 2004/089898, WO 2004/069835, WO 2004/089900 and WO 2004/089364 disclose substituted azabicyclohexane derivatives as muscarinic receptor antagonists. WO 2006/018708 discloses pyrrolidine derivatives as muscarinic receptor antagonists. WO 2006/035303 discloses azabicyclo derivatives as muscarinic receptor antagonists.

WO 01/04118 and WO 2004/096800 disclose quinuclidine derivatives as muscarinic receptors antagonist. WO 2005/090342 discloses quatemized quinuclidine ester as antimuscarinic agents with potent and long lasting effect.

The present invention fills the need of muscarinic receptor antagonists useful in the treatment of disease states with improper smooth muscle function and respiratory disorders.

### Summary of the Invention

In one aspect, there are provided muscarinic receptor antagonists which can be used as therapeutic or prophylactic agents for the treatment of various diseases of the respiratory, urinary and gastrointestinal systems. Also provided are processes for the synthesis of such compounds.

In another aspect, pharmaceutical compositions containing such compounds are provided which may also contain acceptable carriers, excipients or diluents which are useful for the treatment of various diseases of the respiratory, urinary and gastrointestinal systems. The enantiomers, diasteromers, *N*-oxides, polymorphs or solvates and pharmaceutically acceptable solvates of these compounds are also provided, as well as pharmaceutical composition comprising the compounds, their enantiomers, diastereomers, *N*-oxides, polymorphs or solvates thereof, in combination with a pharmaceutically acceptable carrier and optionally included excipients.

In still another aspect, pharmaceutical compositions of one or more compounds of Formula I are provided with at least one other active ingredients selected from corticosteroids, beta agonists, leukotriene antagonists, 5-lipoxygenase inhibitors, anti-histamines, antitussives, dopamine receptor antagonists, chemokine inhibitors, p38 MAP Kinase inhibitors and PDE-IV inhibitors.

Other aspects will be set forth in the description which follows, and in part will be apparent from the description or may be learnt by the practice of the invention.

Detailed Description of the Invention

The present invention relates to compounds having the structure of Formula I

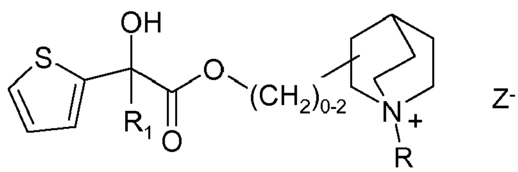

**Formula I**

and their pharmaceutically acceptable solvates, enantiomers, diastereomers, polymorphs or *N*-oxides wherein

$R_1$ is thienyl, or cycloalkyl substituted with difluoro on same carbon (geminal substitution);

R is $C_{1-4}$ linear alkyl or alkyl which is substituted with phenyl or phenyl carbonyl, where phenyl is optionally substituted with one or more halogen atoms, hydroxyl, alkyl, alkenyl, alkynyl, cycloalkyl, alkoxy, acyl, aryloxy, cyano, nitro, $-COOR_c$,$-NHC(=O)R_b$, $-NR_bR_d$, $-C(=O)NR_bR_d$, $-NHC(=O)NR_bR_d$, $-OC(=O)NR_bR_d$, $-SO_mR_c$, heterocyclyl, heteroaryl, heterocyclylalkyl, heteroarylalkyl, $-SR_b$ or haloalkyl; wherein
$R_b$ and $R_d$ are independently selected from hydrogen, alkyl, alkenyl, alkynyl, alkoxy, cycloalkyl, aryl, aralkyl, heterocyclyl, heteroaryl, heterocyclylalkyl, heteroarylalkyl, or carboxy;
$R_c$ is hydrogen, alkyl, alkenyl, alkynyl, cycloalkyl, aralkyl, aryl, heterocyclyl, heteroaryl, heteroarylalkyl or heterocyclylalkyl;
m is an integer from 0 to 2;

Z is an anion selected from acetate, succinate, maleate, methanesulphonate, benzenesulphonate, trifluoroacetate, oxalate, tartarate, citrate, glutamate, fumarate, malonate, adepate, ascorbate, carbonate, camphoenate acid, nicotinate, butyrate, tartarate, lactate, sulphate, phosphate, glucurinate, chloride, bromide, iodide, hexafluorophosphate, nitrate, borate and perchlorate.

In one embodiment, $R_1$ is thienyl, 3, 3-difluorocylopentyl or 3,3-difluorocyclohexyl.

In another embodiment, R is methyl, ethyl, propyl, butyl, benzyl, 4-bromobenzyl, 3 fluorobenzyl, 3,5-difluorobenzyl, 3-bromobenzyl, 4-fluorobenzyl, 4-chlorobenzyl, 2,5-difluorobenzyl, 1-phenylethanone, ethylbenzene or 1-(4-fluorophenyl) ethanone.

In another embodiment, the invention encompasses compounds of Formula I, which may include, but not limited to the following:

(3*R*)-3- {[Hydroxy(dithiophen-2-yl)acetyl]oxy}-1-methyl-1-azoniabicyclo[2.2.2]octane bromide (Compound No. 1);

(3*R*)-1-Benzyl-3-{[hydroxy(dithiophen-2-yl)acetyl]oxy}-1-azoniabicyclo[2.2.2]octane bromide (Compound No. 2);

*(*3*R)*-1-(4-Bromobenzyl)-3-{[hydroxy(dithiophen-2-yl)acetyl]oxy}-1-azoniabicyclo[2.2.2]octane bromide (Compound No. 3);

*(*3*R)*-1-Ethyl-3- {[hydroxy(dithiophen-2-yl)acetyl]oxy}-1-azoniabicyclo[2.2.2]octane bromide (Compound No. 4);

*(*3*R)*-1-(3-Fluorobenzyl)-3-{[hydroxy(dithiophen-2-yl)acetyl]oxy}-1-azoniabicyclo[2.2.2]octane bromide (Compound No. 5);

*(*3R*)*-1-(3,5-Difluorobenzyl)-3-{[hydroxy(dithiophen-2-yl)acetyl]oxy}-1-azoniabicyclo[2.2.2]octane bromide (Compound No. 6);

*(*3*R)*-1-(3-Bromobenzyl)-3-{[hydroxy(dithiophen-2-yl)acetyl]oxy}-1-azoniabicyclo[2.2.2]octane bromide (Compound No. 7);

*(*3*R)*-1-(4-Fluorobenzyl)-3-{[hydroxy(dithiophen-2-yl)acetyl]oxy}-1-azoniabicyclo[2.2.2]octane bromide (Compound No. 8);

*(*3*R)*-1-(4-Chlorobenzyl)-3-{[hydroxy(dithiophen-2-yl)acetyl]oxy}-1-azoniabicyclo[2.2.2]octane bromide (Compound No. 9);

*(*3*R)*-1-(2,5-Difluorobenzyl)-3-{[hydroxy(dithiophen-2-yl)acetyl]oxy}-1-azoniabicyclo[2.2.2]octane bromide (Compound No. 10);

*(*3*R)*-3-{[Hydroxy(dithiophen-2-yl)acetyl]oxy}-1-(2-oxo-2-phenylethyl)-1-azoniabicyclo [2.2.2]octane bromide (Compound No. 11);

*(*3*R)*-3-{[Hydroxy(dithiophen-2-yl)acetyl]oxy}-1-propyl-1-azoniabicyclo[2.2.2]octane bromide (Compound No. 12);

*(*3*R)*-1-Butyl-3-{[hydroxy(dithiophen-2-yl)acetyl]oxy}-1-azoniabicyclo[2.2.2]octane bromide (Compound No. 13);

*(*3*R)*-3-{[(3,3-Difluorocyclopentyl)(hydroxy)thiophen-2-ylacetyl]oxy}-1-methyl-1-zoniabicyclo[2.2.2]octane bromide (Compound No. 14);

*(*3*R)*-3-{[(3,3-Difluorocyclopentyl)(hydroxy)thiophen-2-ylacetyl]oxy}-1-ethyl-1-azoniabicyclo[2.2.2]octane bromide (Compound No. 15);

*(*3*R)*-3-{(3,3-Difluorocyclopentyl)(hydroxy)thiophen-2-ylacetyl]oxy}-1-propyl-1-azoniabicyclo[2.2.2]octane bromide (Compound No. 16);

*(*3*R)*-1-Butyl-3-{[(3,3-difluorocyclopentyl)(hydroxy)thiophen-2-ylacetyl]oxy}-1-azoniabicyclo[2.2.2]octane bromide (Compound No. 17);

*(*3*R)*-3-{[(3,3-Difluorocyclopentyl)(hydroxy)thiophen-2-ylacetyl]oxy}-1-(2-oxo-2-phenylethyl)-1-azoniabicyclo[2.2.2] octane bromide (Compound No. 18);

*(*3*R)*-3-{[(3,3-Difluorocyclopentyl)(hydroxy)thiophen-2-ylacetyl]oxy}-1-(2-phenylethyl)-1-azoniabicyclo[2.2.2]octane bromide (Compound No. 19); and

*(*3*R)*-3-{[(3,3-Difluorocyclopentyl)(hydroxy)thiophen-2-ylacetyl]oxy}-1-[2-(3-fluorophenyl)-2-oxoethyl]-1-azoniabicyclo [2.2.2] octane bromide (Compound No. 20).

In another embodiment, there are provided herein pharmaceutical compositions comprising a therapeutically effective amount of a compound of Formula I described herein together with one or more pharmaceutically acceptable carrier(s), excipients(s) or diluent(s).

In yet another embodiment there are provided methods for the treatment or prophylaxis of a mammal suffering from a disease or disorder of the respiratory, urinary or gastrointestinal systems, wherein the disease or disorder is mediated through muscarinic receptors. The method includes administration of at least one compound having the structure of Formula I.

In yet another embodiment, there are provided methods for the treatment or prophylaxis of a mammal suffering from a disease or disorder of the respiratory system such as bronchial asthma, chronic obstructive pulmonary disorders (COPD), pulmonary fibrosis, and the like; urinary system which induce such urinary disorders as urinary incontinence, lower urinary tract symptoms (LUTS); and gastrointestinal system such as irritable bowel syndrome, obesity, diabetes and gastrointestinal hyperkinesis with compounds of Formula I, wherein the disease or disorder is associated with muscarinic receptors.

In yet another embodiment, there are provided pharmaceutical compositions comprising one or more muscarinic receptor antagonist compound having the structure of Formula I and at least one or more therapeutic agent selected from histamine antagonists, corticosteroids, beta agonists, leukotriene antagonists, EGFR kinase inhibitors, PAF antagonist, 5-lipoxygenase inhibitors, chemokine inhibitors, PDE-4 inhibitors or p-38 MAP kinase inhibitors.

In yet another embodiment, there are provided processes for preparing the compounds of Formula I.

In yet another embodiment, the enantiomeric compounds of the invention may be obtained (a) by separation of the components of the corresponding racemic mixture, for example by preferential crystallization using chiral resolving agents, chiral chromatography, enzymatic resolution methods or preparing and separating suitable diastereoisomers, (b) by direct synthesis from the appropriate chiral starting materials or by using chiral reagents.

The term "alkyl," unless otherwise specified, refers to a monoradical branched or unbranched saturated hydrocarbon chain having from 1 to 20 carbon atoms. Alkyl groups can be optionally interrupted by atom(s) or group(s) independently selected from oxygen, sulfur, a phenylene, sulphinyl, sulphonyl group or $-NR_\alpha-$, wherein $R_\alpha$ can be hydrogen, alkyl, cycloalkyl, alkenyl, cycloalkenyl, alkynyl, aryl, acyl, aralkyl, $-C(=O)OR_\lambda$, $SO_mR_\psi$ or $-C(=O)NR_\lambda R_\pi$. This term can be exemplified by groups such as methyl, ethyl, n-propyl, iso-propyl, n-butyl, iso-butyl, sec-butyl, t-butyl, n-pentyl, isopentyl, neopentyl, n-hexyl, n-decyl, tetradecyl, and the like. Alkyl groups may be substituted further with one or more substituents selected from alkenyl, alkynyl, alkoxy, cycloalkyl, cycloalkenyl, acyl, acylamino, acyloxy, alkoxycarbonylamino, azido, cyano, halogen, hydroxy, keto, oxo, thiocarbonyl, substituted thiocarbonyl, carboxy, carboxyalkyl, aryl, heterocyclyl, heteroaryl, (heterocyclyl)alkyl, cycloalkoxy, $-NR_\lambda C(=O)OR_\lambda$, $COOR_\lambda$, $-CH=N-O(C_{1-6}alkyl)$, $-CH=N-NH(C_{1-6}\ alkyl)$, $-CH=N-NH(C_{1-6}\ alkyl)-C_{1-6}$ alkyl, arylthio, thiol, alkylthio, aryloxy, alkoxyamino, nitro, aminosulfonyl, aminocarbonylamino, $-NHC(=O)R_\lambda$, $-NR_\lambda R_\pi$, $-C(=O)NR_\lambda R_\pi$, $-NR_\lambda C(=O)NR_\lambda R_\pi$, -C(=O)heteroaryl, C(=O)heterocyclyl, $-O-C(=O)NR_\lambda R_\pi$ {wherein $R_\lambda$ and $R_\pi$ are independently selected from hydrogen, halogen, hydroxy, alkyl, alkenyl, alkynyl, alkoxy, cycloalkyl, cycloalkenyl, aryl, aralkyl, heterocyclyl, heteroaryl, heterocyclylalkyl, heteroarylalkyl, carboxy or $R_\lambda$ and $R_\pi$ may also together join to form a heterocyclyl or heteroaryl ring} or $-SO_mR_\psi$ (wherein m is an integer from 0-2 and $R_\psi$ is hydrogen, alkyl, alkenyl, alkynyl, cycloalkyl, aralkyl, aryl, heterocyclyl, heteroaryl, heteroarylalkyl or heterocyclylalkyl). Unless otherwise constrained by the definition, alkyl substituents may be further substituted by 1-3 substituents selected from alkyl, alkenyl, alkynyl, carboxy, $-NR_\lambda R_\pi$, $-C(=O)NR_\lambda R_\pi$, $-OC(=O)NR_\lambda R_\pi$, $-NR_\lambda C(=O)NR_\lambda R_\pi$, hydroxy, alkoxy, halogen, $CF_3$, cyano, and $-SO_mR_\psi$; or an alkyl group also may be interrupted by 1-5 atoms of groups independently selected from oxygen, sulfur or $-NR_\alpha-$(wherein $R_\alpha$, $R_\lambda$, $R_\pi$, m and $R_\psi$ are the same as defined earlier). Unless otherwise constrained by the definition, all substituents may be substituted further by 1-3 substituents selected from alkyl, alkenyl, alkynyl, carboxy, carboxyalkyl, $-NR_\lambda R_\pi$, $-C(=O)NR_\lambda R_\pi$, $-O-C(=O)NR_\lambda R_\pi$, hydroxy, alkoxy, halogen, $CF_3$, cyano, and $-SO_mR_\psi$ (wherein $R_\lambda$, $R_\pi$, m and $R_\psi$ are the same as defined earlier); or an alkyl group as defined above that has both substituents as defined above and is also interrupted by 1-5 atoms or groups as defined above.

The term "alkenyl," unless otherwise specified, refers to a monoradical of a branched or unbranched unsaturated hydrocarbon group having from 2 to 20 carbon atoms with cis, trans or geminal geometry. Alkenyl groups can be optionally interrupted by atom(s) or group(s) independently chosen from oxygen, sulfur, phenylene, sulphinyl, sulphonyl and $-NR_\alpha-$ (wherein $R_\alpha$ is the same as defined earlier). In the event that alkenyl is attached to a heteroatom, the double bond cannot be alpha to the heteroatom. Alkenyl groups may be substituted further with one or more substituents selected from alkyl, alkenyl, alkynyl, alkoxy, cycloalkyl, cycloalkenyl, acyl, acylamino, acyloxy, $-NHC(=O)R_\lambda$, $-NR_\lambda R_\pi$, $-C(=O)NR_\lambda R_\pi$, $-NHC(=O)NR_\lambda R_\pi$, $-O-C(=O)NR_\lambda R_\pi$, alkoxycarbonylamino, azido, cyano, halogen, hydroxy, oxo, keto, carboxyalkyl, thiocarbonyl, carboxy, arylthio, thiol, alkylthio, aryl, aralkyl, aryloxy, heterocyclyl, heteroaryl, heterocyclylalkyl, heteroarylalkyl, aminosulfonyl, aminocarbonylamino, alkoxyamino, hydroxyamino, alkoxyamino, nitro or $SO_mR_\psi$ (wherein $R_\lambda$, $R_\pi$, m and $R_\psi$ are as defined earlier). Unless otherwise constrained by the definition, alkenyl substituents optionally may be substituted further by 1-3 substituents selected from alkyl, alkenyl, alkynyl, carboxy, hydroxy, alkoxy, halogen, $-CF_3$, cyano, $-NR_\lambda R_\pi$, $-C(=O)NR_\lambda R_\pi$, $-O-C(=O)NR_\lambda R_\pi$ and $-SO_mR_\psi$ (wherein $R_\lambda$, $R_\pi$, m and $R_\psi$ are as defined earlier). Groups, such as ethenyl or vinyl ($CH=CH_2$), 1-propylene or allyl ($-CH_2CH=CH_2$), iso-propylene ($-C(CH_3)=CH_2$), bicyclo[2.2.1]heptene, and the like, exemplify this term.

The term "alkynyl," unless otherwise specified, refers to a monoradical of an unsaturated hydrocarbon, having from 2 to 20 carbon atoms. Alkynyl groups can be optionally interrupted by atom(s) or group(s) independently chosen from oxygen, sulfur, phenylene, sulphinyl, sulphonyl and $-NR_\alpha-$ (wherein $R_\alpha$ is the same as defined earlier). In the event that alkynyl groups are attached to a heteroatom, the triple bond cannot be alpha to the heteroatom. Alkynyl groups may be substituted further with one or more substituents selected from alkyl, alkenyl, alkoxy, cycloalkyl, cycloalkenyl, acyl, acylamino, acyloxy, alkoxycarbonylamino, azido, cyano, halogen, hydroxy, keto, oxo, thiocarbonyl, carboxy, carboxyalkyl, arylthio, thiol, alkylthio, aryl, aralkyl, aryloxy, aminosulfonyl, aminocarbonylamino, hydroxyamino, alkoxyamino, nitro, heterocyclyl, heteroaryl, heterocyclylalkyl, heteroarylalkyl, NHC(=O)$R_\lambda$, -N$R_\lambda R_\pi$, -NHC(=O)N$R_\lambda R_\pi$, -C(=O)N$R_\lambda R_\pi$, -O-C(=O)N$R_\lambda R_\pi$ or -$SO_m R_\psi$ (wherein $R_\lambda$, $R_\pi$, m and $R_\psi$ are the same as defined earlier). Unless otherwise constrained by the definition, alkynyl substituents optionally may be substituted further by 1-3 substituents selected from alkyl, alkenyl, alkynyl, carboxy, carboxyalkyl, hydroxy, alkoxy, halogen, $CF_3$, -N$R_\lambda R_\pi$, -C(=O)N$R_\lambda R_\pi$, -NHC(=O)N$R_\lambda R_\pi$, -C(=O)N$R_\lambda R_\pi$, cyano or -$SO_m R_\psi$ (wherein $R_\lambda$, $R_\pi$, m and $R_\psi$ are the same as defined earlier).

The term "cycloalkyl," unless otherwise specified, refers to cyclic alkyl groups of from 3 to 20 carbon atoms having a single cyclic ring or multiple condensed rings, which may optionally contain one or more olefinic bonds, unless otherwise constrained by the definition. Such cycloalkyl groups can include, for example, single ring structures, including cyclopropyl, cyclobutyl, cyclopentyl, cyclohexyl, cycloheptyl, cyclooctyl, cyclopentenyl, and the like or polycyclic ring structures such as, adamantyl, tricyclo[3.3.1.1]decane, bicyclo[2.2.2]octane, bicyclo[4.4.0]decane, bicyclo-[4.3.0]nonane, bicyclo[3.3.0]octane, bicyclo[2.2.1]heptane and the like, or cyclic alkyl groups to which is fused an aryl group, for example, indane, and the like. Spiro and fused ring structures can also be included. Cycloalkyl groups may be substituted further with one or more substituents selected from alkyl, alkenyl, alkynyl, alkoxy, cycloalkyl, cycloalkenyl, acyl, acylamino, acyloxy, alkoxycarbonylamino, azido, cyano, halogen, hydroxy, oxo, thiocarbonyl, carboxy, carboxyalkyl, arylthio, thiol, alkylthio, aryl, aralkyl, aryloxy, aminosulfonyl, aminocarbonylamino, -N$R_\lambda R_\pi$, -NHC(=O)N$R_\lambda R_\pi$, -NHC(=O)$R_\lambda$, -C(=O)N$R_\lambda R_\pi$, -O-C(=O)N$R_\lambda R_\pi$, nitro, heterocyclyl, heteroaryl, heterocyclylalkyl, heteroarylalkyl or $SO_m R_\psi$ (wherein $R_\lambda$, $R_\pi$, m and $R_\psi$ are the same as defined earlier). Unless otherwise constrained by the definition, cycloalkyl substituents optionally may be substituted further by 1-3 substituents selected from alkyl, alkenyl, alkynyl, carboxy, hydroxy, alkoxy, halogen, $CF_3$, -N$R_\lambda R_\pi$, -C(=O)N$R_\lambda R_\pi$, -NHC(=O)N$R_\lambda R_\pi$, -OC(=O)N$R_\lambda R_\pi$, cyano or -$SO_m R_\psi$ (wherein $R_\lambda$, $R_\pi$, m and $R_\psi$ are the same as defined earlier). "Cycloalkylalkyl" refers to alkyl-cycloalkyl group linked through alkyl portion, wherein the alkyl and cycloalkyl are the same as defined earlier.

The term "alkoxy" denotes the group O-alkyl, wherein alkyl is the same as defined above.

The term "aryl," unless otherwise specified, refers to aromatic system having 6 to 14 carbon atoms, wherein the ring system can be mono-, bi- or tricyclic and are carbocyclic aromatic groups. For example, aryl groups include, but are not limited to, phenyl, biphenyl, anthryl or naphthyl ring and the like, optionally substituted with 1 to 3 substituents selected from halogen (e.g., F, Cl, Br, I), hydroxy, alkyl, alkenyl, alkynyl, cycloalkyl, alkoxy, acyl, aryloxy, $CF_3$, cyano, nitro, -CHO, $OCF_3$, -$SCF_3$, COO$R_\psi$, NHC(=O)$R_\lambda$, -N$R_\lambda R_\pi$, C(=NOH)$NH_2$, -C(=O)NR$R_\lambda R_\pi$, -N$R_\lambda$C(=O)N$R_\lambda R_\pi$, N$R_\lambda$C(=O)O$R_\lambda$, -O-C(=O)N$R_\lambda R_\pi$, -$SO_m R_\psi$, carboxy, heterocyclyl, heteroaryl, heterocyclylalkyl, heteroarylalkyl, acylamino, thiocarbonyl, substituted thiocarbonyl, amino carbonyl amino, mercapto, haloalkyl, optionally substituted aryl, optionally substituted heterocyclylalkyl, thioalkyl, -CONH$R_\pi$, -OCO$R_\pi$, -CO$R_\pi$, -NH$SO_2 R_\pi$ or -$SO_2$NH$R_\pi$ (wherein $R_\lambda$, $R_\pi$, m and $R_\psi$ are the same as defined earlier). Aryl groups optionally may be fused with a cycloalkyl group or a heteroaryl group, wherein the cycloalkyl group may optionally contain heteroatoms selected from O, N or S. Groups such as phenyl, naphthyl, anthryl, biphenyl, and the like exemplify this term.

The term "aralkyl," unless otherwise specified, refers to alkyl-aryl linked through an alkyl portion (wherein alkyl is as defined above) and the alkyl portion contains 1-6 carbon atoms and aryl is as defined below. Examples of aralkyl groups include benzyl, ethylphenyl, propylphenyl, naphthylmethyl and the like.

The term "aryloxy" denotes the group O-aryl, wherein aryl is as defined above.

The term "carboxy," as defined herein, refers to -C (=O) OH.

The term "heteroaryl," unless otherwise specified, refers to an aromatic monocyclic, bicyclic or a tricyclic ring system (they can be fused, spiro or bridged) containing 1-8 heteroatom(s) independently selected from N, O or S optionally substituted with 1 to 4 substituent(s) selected from halogen (e.g., F, Cl, Br, 1), hydroxy, alkyl, alkenyl, alkynyl, cycloalkyl, acyl, acylamino, thiocarbonyl, substituted thiocarbonyl, thioacyl, oxo, -CHO, -$OCF_3$, -$CF_3$, -$SCF_3$, carboxy, aryl, alkoxy, alkoxyamino, aralkyl, cyano, nitro, heterocyclyl, heteroaryl, -N$R_\lambda R_\pi$, CH=NOH, -$(CH_2)_w$C(=O)$R_\eta$, COO$R_\lambda$ {wherein w is an integer from 0-4 and $R_\eta$ is hydrogen, hydroxy, O$R_\lambda$, N$R_\lambda R_\pi$, -NHO$R_\omega$ or -NHOH}, -C(=O)N$R_\lambda R_\pi$, -N$R_\lambda$C(=O)O$R_\lambda$, -N$R_\lambda$C(=O)N$R_\lambda R_\pi$, -$SO_m R_\psi$, -O-C(=O)N$R_\lambda R_\pi$, -O-C(=O)$R_\lambda$, or -O-C(=O)O$R_\lambda$ (wherein m, $R_\psi$, $R_\lambda$ and $R_\pi$ are as defined earlier and $R_\omega$ is alkyl, cycloalkyl, aryl, heteroaryl, heterocyclyl, heteroarylalkyl or heterocyclylalkyl). Unless otherwise constrained by the definition, the substituents are attached to a ring atom, i.e., carbon or heteroatom in the ring. Examples of heteroaryl groups includes but are not limited to are benzimidazolyl, 1,4-benzodioxanyl, 1,3-benzodioxolyl, benzoxazolyl, benzothiazolyl, benzothienyl, benzo- triazolyl, dihydroimidazolyl, dihydropyranyl, dihydrofuranyl, dioxanyl, dioxolanyl, furyl, homopiperidinyl, imidazolyl, imidazolinyl, imidazolidinyl, indolinyl, indolyl, isoquinolinyl, isothiazolidinyl, isothiazolyl, isoxazolidinyl, isoxazolyl, morpholinyl, napthyridinyl, oxazolidinyl, oxazolyl, piperazinyl, piperidinyl, purinyl, pyrazinyl, pyrazolinyl, pyridinyl, pyrimidinyl, pyrrolidinyl, pyrrolinyl, pyrrolyl, pyrrolopyridinyl, imidazolpyridinyl, quinolinyl, tetrahydrofuranyl, quinozinyl, quinolizinyl, 6*H*-pyrido-[1,2-*a*]pyrimidinyl, tetrahydropyranyl, thiazolidinyl, thiazolyl, thienyl, pyridazinyl, carbazolyl, isobenzofuranyl, thianthrene, triazinyl, furanyl, benzofuranyl, tetrazolyl, quinazolinyl, benzoxazinonyl, benzothiazinonyl, benzimidazolone, pyrazolone, xanthene and the like.

The term "halogen or halo" refers to fluorine, chlorine, bromine or iodine.

The term "haloalkyl" refers to alkyl of which one or more hydrogen(s) is/are replaced by halogen.

The term "heterocyclyl," unless otherwise specified, refers to a non-aromatic monocyclic or polycyclic ring (fused, spiro or bridged) system having 1 to 8 heteroatoms selected from O, S or N, and optionally are benzofused or fused heteroaryl having 5-6 ring members and/or optionally substituted, wherein the substituents are selected from halogen (e.g., F, Cl, Br, I), hydroxy, alkyl, alkenyl, alkynyl, cycloalkyl, acyl, acylamino, optionally substituted thiocarbonyl, optionally substituted aryl, alkoxy, alkoxyamino, alkaryl, cyano, nitro, oxo, -CHO, $-OCF_3$, $-CF_3$, $-SCF_3$, carboxy, optionally substituted heterocyclyl, optionally substituted heterocyclylalkyl, optionally substituted heteroaryl, $-O-C(=O)R_\lambda$, $-O-C(=O)OR_\lambda$, $-C(=O)NR_\lambda R_\pi$, $SO_mR_\psi$, $-O-C(=O)NR_\lambda R_\pi$, $-NR_\lambda C(=O)OR_\lambda$, $-NR_\lambda C(=O)NR_\lambda R_\pi$, $-NR_\lambda R_\pi$, mercapto, haloalkyl, thioalkyl, $-COOR_\psi$, $-COONHR_\lambda$, $-COR_\lambda$, $-NHSO_2R_\lambda$ or $SO_2NHR_\lambda$ (wherein m, $R_\psi$, $R_\lambda$ and $R_\pi$ are as defined earlier) or guanidine. Heterocyclyl can optionally include rings having one or more double bonds. Such ring systems can be mono-, bi- or tricyclic. Carbonyl or sulfonyl group can replace carbon atom(s) of heterocyclyl. Unless otherwise constrained by the definition, the substituents are attached to the ring atom, i.e., carbon or heteroatom in the ring. Also, unless otherwise constrained by the definition, the heterocyclyl ring optionally may contain one or more olefinic bond(s). Examples of heterocyclyl groups includes but are not limited to are tetrahydrofuranyl, dihydrofuranyl, dihydropyridinyl, dihydrobenzofuryl, azabicyclohexyl, dihydroindolyl, piperidinyl, isoxazolinyl, thiazolinyl, thiazolidinonyl, oxazolinyl, oxazolidinonyl, azabicyclo[3.1.0]hexyl, diazabicyclo[2.2.1]heptyl, azetidinyl, 1,4-benzodioxanyl, 1,3-benzodioxolyl, dihydrobenzofuryl, dihydroimidazolyl, dihydropyranyl, dihydrofuranyl, dihydroindolyl, dihydroisoxazolyl, dihydropyridinyl, dioxanyl, dioxolanyl, homopiperi-dinyl, imidazolinyl, imidazolidinyl, imidazopyridinyl, indolinyl, indolyl, isoindole1,3-dione, isothiazolidinyl, morpholinyl, napthyridinyl, oxazolidinyl, oxazolyl, phenoxazinyl, phenothiazinyl, piperazinyl, purinyl, pyrazinyl, pyrazolinyl, pyrazolyl, pyrimidinyl, pyrrolidinyl, pyrrolinyl, pyrrolyl, pyrrolopyridinyl, tetrahydropyranyl, tetrazolyl, thiazolidinyl and thiazolyl, and thienyl and the like.

"Heteroarylalkyl" refers to alkyl-heteroaryl group linked through alkyl portion, wherein the alkyl and heteroaryl are as defined earlier.

"Heterocyclylalkyl" refers to alkyl-heterocyclyl group linked through alkyl portion, wherein the alkyl and heterocyclyl are as defined earlier.

"Acyl" refers to $-C(=O)R_{¢¢}$ wherein $R_{¢¢}$ is selected from hydrogen, alkyl, cycloalkyl, aryl, aralkyl, heteroaryl, heterocyclyl, heteroarylalkyl or heterocyclylalkyl.

The phrase "pharmaceutically acceptable carriers" is intended to include non-toxic, inert solid, semi-solid or liquid filler, diluent, encapsulating material or formulation auxiliary of any type.

The term "Protecting Groups" is used herein to refer to known moieties, which have the desirable property of preventing specific chemical reaction at a site on the molecule undergoing chemical modification intended to be left unaffected by the particular chemical modification. Also the term protecting group, unless or other specified may be used with groups such as hydroxy, amino, carboxy and example of such groups are found in T.W. Greene and P.G.M. Wuts, "Protective Groups in Organic Synthesis", 2nd Edn. John Wiley and Sons, New York, N.Y., which is incorporated herein by reference. The species of the carboxylic protecting groups, amino protecting groups or hydroxy protecting group employed is not so critical so long as the derivatised moiety/moieties is/are stable to conditions of subsequent reactions and can be removed at the appropriate point without disrupting the remainder of the molecule.

The term "pharmaceutically acceptable salts" refers to derivatives of compounds that can be modified by forming their corresponding acid or base salts. Pharmaceutically acceptable salts may also be formed by complete derivatization of the amine moiety, *e.g.*, quaternary ammonium salts.

The compounds described herein exhibit affinity for $M_3$ receptor, as determined by *in vitro* receptor binding assay. Pharmaceutical compositions for the possible treatment for the disease or disorders associated with muscarinic receptors are provided. In addition, the compounds can be administered orally or parenterally.

The compounds disclosed herein may be prepared by methods represented by the reaction sequences, shown in Scheme I.

Scheme I

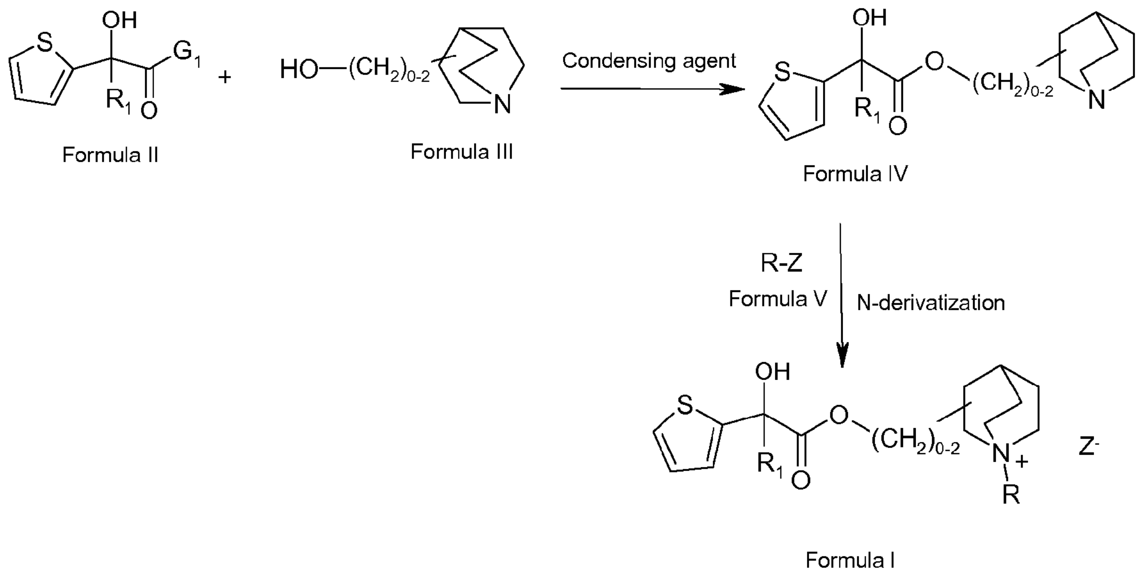


The compounds of Formula I can be prepared following the procedure as depicted in Scheme I wherein the reaction comprises condensing a compound of Formula II (wherein $R_1$ is the same as defined earlier, $G_1$ is hydroxyl or -Oalkyl) with a compound of Formula III to give a compound of Formula IV. The *N*-derivatization of a compound of Formula IV with compound of Formula V (where R and Z are same as defined earlier) to give compound of Formula I.

The condensation of a compound of Formula II with a compound of Formula III to give a compound of Formula IV can be carried out in an organic solvent (for example toluene, benzene, hexane, xylene, diethyl ether, dichloromethane, chloroform or carbon tetrachloride or mixture(s) thereof) in the presence of base (for example sodium metal, sodium alkoxide, lithium hydride, triethylamine, *N*-ethyldiisopropylamine, pyridine, potassium alkoxide).

Alternatively, condensation of a compound of Formula II with a compound of Formula III to give a compound of Formula IV can be carried out in an organic solvent (for example dimethylformamide, dichloromethane, carbon tetrachloride, chloroform, tetrahydrofuran, diethylether or dioxane) in the presence of base (for example *N*-methylmorpholine, triethyamine, diisopropylethylamine or pyridine) with a condensing agent (for example, 1-(3-dimethylaminopropyl)-3-ethylcarbodiimide hydrochloride (EDC.HCl) or dicyclohexylcarbodiimide (DCC)).

*N*-derivatization of a compound of Formula IV with compound Formula V to give a compound of Formula I can be carried out in an organic solvent (for example, acetonitrile, dichloromethane, dimethylformamide, tetrahydrofuran, chloroform, carbon tetrachloride or mixture(s) thereof).

Compounds Nos. 1-20 were prepared following Scheme I.

In the above schemes, where specific bases, condensing agents, resolving agents, protecting groups, deprotecting agents, solvents, catalysts, temperatures, etc. are mentioned, it is to be understood that other bases, condensing agents, resolving agents, protecting groups, deprotecting agents, solvents, catalysts, temperatures, etc. known to those skilled in the art may be used. Similarly, the reaction temperature and duration may be adjusted according to the desired needs.

The compounds described herein can be produced and formulated as their enantiomers, diastereomers, *N*-Oxides, polymorphs, solvates having the same type of activity. Pharmaceutical compositions comprising the molecules of Formula I or enantiomers, diastereomers, *N*-oxides, polymorphs, solvates thereof, in combination with pharmaceutically acceptable carrier and optionally included excipient can also be produced.

Where desired, the compounds of Formula I and/or their pharmaceutically acceptable solvates, stereoisomers, polymorphs or *N*-oxides may be advantageously used in combination with one or more other therapeutic agents. Examples of other therapeutic agents, which may be used in combination with compounds of Formula I of this invention and/or their pharmaceutically acceptable solvates, stereoisomers, polymorphs or *N*-oxides include but are not limited to, corticosteroids, beta agonists, leukotriene antagonists, 5-lipoxygenase inhibitors, anti-histamines, antitussives, dopamine receptor antagonists, chemokine inhibitors, p38 MAP Kinase inhibitors and PDE-IV inhibitors.

The described compounds have pharmacological activity, and accordingly, may be administered to an animal for oral or parenteral treatment. Pharmaceutical compositions described herein comprise therapeutically effective

amounts of one or more compounds described herein formulated together with one or more pharmaceutically acceptable carriers. The term "pharmaceutically acceptable carriers" is intended to include non-toxic, inert solid, semi-solid or liquid filter, diluent, encapsulating materials or formulation auxiliaries of any type. Solid form preparations for oral administrations include, but are not limited to, capsules, tablets, pills, powders, granules, or suppositories. For solid form preparations, active compounds can be mixed with one or more inert, pharmaceutically acceptable excipient or carrier, for example, sodium citrate, dicalcium phosphate and/or fillers or extenders (for example, starch, lactose, sucrose, glucose, mannitol, silicic acid or mixtures thereof); binders, for example, carboxymethylcellulose, alginates, gelatins, polyvinylpyrolidinone, sucrose, acacia or mixtures thereof; disintegrating agents, for example, a agar-agar, calcium carbonate, potato starch, alginic acid, silicates, sodium carbonate or mixtures thereof; absorption accelerators, for example, quaternary ammonium compounds; wetting agents, for example, cetyl alcohol, glycerol, monostearate or mixtures thereof; adsorbents, for example, kaolin; lubricants, for example, talc, calcium stearate, magnesium stearate, solid polyethyleneglycol, sodium lauryl sulfate, or mixtures thereof; or mixtures thereof.

For capsules, tablets, or pills, the dosage form also may comprise one or more buffering agents. Solid preparations of tablets, capsules, pills or granules can be prepared using one or more coatings or shells, for example, enteric coatings and other coatings well known to one of ordinary skill in the art.

Liquid form preparations for oral administration can include pharmaceutically acceptable emulsions, solutions, suspensions, syrups or elixirs. For liquid form preparations, active compounds can be mixed with water or one or more other solvents, solubilizing agents or emulsifiers, for example, ethyl alcohol, isopropyl alcohol, ethyl carbonate, ethyl acetate, benzyl alcohol, benzyl benzoate, propylene glycol, 1,3-butylene glycol, dimethylformamide, oils (for example, cottonseed, groundnut, corn, germ, olive, castor, sesame oil or mixtures thereof), glycerol, and fatty acid esters of sorbitan or mixtures thereof. )Oral compositions can also include one or more adjuvants, for example, wetting agents, emulsifying agents, suspending agents, sweetening agents, flavoring agents, perfuming agents or mixtures thereof.

Pharmaceutical preparations may be in unit dosage form. In unit dosage form, the preparation may be subdivided into unit doses containing appropriate quantities of active components. Unit dosage forms can be packaged preparations, the package containing discrete capsules, powders, in vials or ampoules and ointments, capsules, cachets, tablets, gel creams or any combination and number of any packaged forms.

Formulations described herein may be formulated so as to provide immediate, sustained, or delayed release of active ingredients after administration to a patient by employing procedures well known to one of ordinary skill in the art.

While the present invention has been described in terms of its specific embodiments, certain modification and equivalents will be apparent to those skilled in the art and are included within the scope of the present invention. The examples are provided to illustrate particulars aspects of the disclosure and do not limit the scope of the present invention as defined by the claims.

Experimental Details

Various solvents were dried using drying reagents according to procedures described in the literature. Wherever room temperature is mentioned it stands for 25-30 °C. Enantiomers were separated using various chiral resolving agents.

Synthesis of ethyl hydroxy (dithiophen-2-yl)acetate

This compound was synthesized as per the procedure in J. Am. Chem. Soc. (1951), 73, 2216-2218.

Synthesis of (2R,1R+2S,1S) or (2R,1S+2S,1R)-(3,3-difluorocyclopentyl) (hydroxyl) thiophen-2-ylacetic acid

**Step a: Synthesis of ethyl oxo(thiophen-2-yl)acetate**

To the solution of thiophene (0.2376 mol, 20 gm) in dichloromethane (about 600 ml) at 0 to -5°C was added ethyl chloro oxoacetate (0.2376 mol, 32.4 gm) dropwise. After addition, the reaction mixture was stirred at this same temperature for about 20 minutes followed by portionwise addition of anhydrous aluminum chloride (0.2376 mol, 31.7 gm). The reaction mixture was stirred at this same temperature for about one hour. The reaction mixture was poured into ice-cooled dilute hydrochloric acid. The two layers were separated and the aqueous layer was extracted with dichloromethane. The combined organic layer was washed with water, brine, dried over anhydrous sodium sulphate and concentrated under reduced pressure and the residue thus obtained was purified by column chromatography. Yield = 25.5 gm.

**Step b: Synthesis of ethyl hydroxy(thiophen-2-yl)acetate**

To a solution of the compound obtained from step *a* above (0.0814 mol, 15 gm) in methanol (about 150 ml) at

0 °C was added sodium borohydride (0.0407 mol, 1.54 gm) portionwise. The reaction mixture was stirred at 25 °C for about one hour. The reaction mixture was concentrated under reduced pressure and the residue thus obtained was taken in ethyl acetate and washed with water, brine and dried over anhydrous sodium sulphate and concentrated under reduced pressure. The residue thus obtained was purified by column chromatography to obtain the title compound. Yield = 13 gm.

**Step c: Synthesis of hydroxy(thiophen-2-yl)acetic acid**

To a solution of the compound obtained from step b above (0.080 mol, 13.63 gm) in methanol (about 200 ml) at 0 °C was added aqueous solution of potassium hydroxide (0.4008 mol, 22.5 gm) drop wise. The reaction mixture was stirred at 25 °C for about 12 hours and was subsequently concentrated under reduced pressure. The residue thus obtained was taken in water, acidified with concentrated hydrochloric acid upto pH ≈1 and extracted with ethyl acetate. The combined organic layer was washed with water, brine, dried over anhydrous sodium sulphate and concentrated under reduced pressure to furnish the title compound. Yield = 12.4 gm.

**Step d: Synthesis of 2-*tert*-butyl-5-thiophen-2-yl-1,3-dioxolan-4-one**

To a solution of the compound obtained from step c above (0.078 mol, 12.12 gm) in benzene (about 300 ml) were added trimethyl acetaldehyde (0.092 mol, 7.92 gm) and *p*-toluene sulphonic acid (0.0015 mol, 291 mg). The reaction mixture was refluxed for about 12 hours and water was removed azeotropically with a Dean-Stark apparatus. The reaction mixture was concentrated under reduced pressure and the residue thus obtained was taken in ethyl acetate and washed with sodium bicarbonate solution, water and brine. The combined organic layer was dried over anhydrous sodium sulphate and concentrated under reduced pressure. The residue thus obtained was purified by column chromatography to yield the two diastereomers. (Diastereomer A and B) of the title compound.
Yield of Diastereomer A (Less polar): 1.5 gm
Yield of Diastereomer B (more polar): 5.3 gm

Taken further 5.3 gm of diastereomer B for reaction which can be mixture of 2*R*, 5*R* + 2*S*, 5*S* (or 2*R*, 5S + 2*S*, 5R).

**Step e: Synthesis of 2-*tert*-butyl-5-[(1'*S* or 1'*R*)-3-oxocyclopentyl]-5-(2-thienyl)-1,3-dioxolan-4-one**

To a solution of the Diastereomer B (0.0296 mol, 6.7 gm) in dry tetrahydrofuran (about 100 ml) at -78 °C under nitrogen atmosphere was slowly added 2 molar solution of lithium diisopropylamide (0.0741 mol, 37 ml). The reaction mixture was stirred at the same temperature for about one hour followed by addition of 0.5 molar solution of zinc chloride in tetrahydrofuran (0.044 mol, 89 ml). The reaction mixture was again stirred at the same temperature for about one hour followed by slow addition of tetramethylethylenediamine (0.5929 mol, 89.0 ml). The reaction mixture was further stirred for about one hour followed by addition of cyclopentenone (0.0325 mol, 2.6 ml). The reaction mixture was stirred at same temperature for about 45 minutes. The reaction mixture was subsequently quenched by slow addition of sulphuric acid (4M, 10 ml) at -78 °C. The remaining 60 ml of sulphuric acid solution was slowly added to reaction mixture at -70 °C. The reaction mixture was again stirred at 25 °C for about 20 minutes and extracted with ethyl acetate. The combined organic layer was washed with aqueous sodium bicarbonate solution, water and brine, dried over sodium sulphate and concentrated under reduced pressure. The residue thus obtained was purified by column chromatography to obtain diastereomer. Yield of Diastereomer B1 (more polar): 2.89 gm can be mixture of 2*R*, 5*R*, 1*R* + 2*S*, 5*S*, 1*S* (or 2*R*, 5*R*, 1*S* + 2*S*, 5*S*, 1*R*).

**Step f: Synthesis of 2-*tert*-butyl-5-[(1'*S or 1'R*)-3,3-difluorocyclopentyl]-5-(2-thienyl)-1,3-dioxolan-4-one**

To a solution of the Diastereomer B1 (0.093 mol, 2.89 gm) in chloroform (10 ml) at 0 °C was added dropwise diethylaminosulphurtrifluoride (0.0281 mol, 3.4 ml). The reaction mixture was stirred at about 25 °C for about two days. The reaction mixture was cooled to 0 °C followed by dropwise addition of aqueous solution of ammonium chloride. The reaction mixture was extracted with dichloromethane. The combined organic layer was washed with water, brine, dried over anhydrous sodium sulphate and concentrated under reduced pressure. The residue so obtained was purified by column chromatography. Yield =1.6 gm. This can be the mixture of 2*R*, 5*R*, 1*R* + 2*S*, 5*S*, 1*S* (or 2*R*, 5*R*, 1*S* + 2*S*, 5*S*, 1*R*).

**Step g: Synthesis of 2R, 1*R*+2*S*, 1*S* or (2R, 1*S*+2*S*, 1*R*)-3, 3-difluorocyclopentyl (hydroxyl) 2-thienylethanoic acid**

To a solution of compound obtained from step *f* above (0.0048 mol, 1.6 gm) in methanol (about 25ml) at 0 °C was added drop wise aqueous sodium hydroxide solution (0.0469 mol, 1.9 gm). The reaction mixture was stirred at 25 °C for about 12 hours and was subsequently concentrated under reduced pressure. The residue thus obtained was

taken in water, acidified with concentrated hydrochloric acid and extracted with ethyl acetate. The combined organic layer was washed with water and brine, dried over anhydrous sodium sulphate and concentrated under reduced pressure to obtain desired compound. Yield = 1.2 gm.
$^1$H NMR $CDCl_3$ δ: 7.28-7.30 (m, 1Ar), 7.14-7.15(m, 1Ar H), 6.96-6.98 (m, 1ArH), 3.06-3.10 (m, 1H), 1.78-2.08 (m, 6H).

Resolution of 1R,2R + 1S,2S or (1R,2S + 1S,2R)-3, 3-difluorocyclopentyl (hydroxyl) 2-thienylethanoic acid

To a solution of (+)-quinidine (3.90 mmol, 1.26 gm) in ethanol (about 25 ml) at 0°C was added 1R,2R+1S,2S or (1R,2S+1S,2R)-3, 3-difluorocyclopentyl (hydroxyl) 2-thienylethanoic acid (1 gm) in ethanol (about 10 ml). The reaction mixture was kept at room temperature for about two days. The crystals so formed was filtered out and dried under vacuum. The crystal thus obtained had 94.22 % chiral purity. The crystal were again dissolved in ethanol and kept at room temperature for about three days. The crystals were separated and dried under vacuum. The enantiomers separated have enantiomeric purity upto 99%. Both enantiomers were isolated, one crystallized out and other present in mother liquor with the enantiomeric purity upto 99%.

Example 1: Synthesis of (3*R*)-3-{[(3,3-difluorocyclopentyl)(hydroxy)thiophen-2-ylacetyl]oxy}-1-methyl-1-azoniabicyclo [2.2.2]octane (Compound No. 14)

**Step a: Synthesis of 1R,2R+1S,2S or (1R,2S+1S,2R)-ethyl (3,3-difluorocyclopentyl) (hydroxy)thiophen-2-ylacetate**

To a solution of 1R,2R+1S,2S or (1R,2S+1S,2R)-(3,3-difluorocyclopentyl)(hydroxyl)thiophen-2-ylacetic acid (0.0007 mol, 200 mg) in dimethylformamide (about 2ml) was added sodium bicarbonate (0.0009 mol, 83 mg) followed by ethyl iodide (0.0014 mol, 0.08 ml). The reaction mixture was stirred at 25 °C for about 12 hours. The reaction mixture was quenched with water and extracted with ethyl acetate. The combined organic layer was washed with water, brine and dried over anhydrous sodium sulphate and concentrated under reduced pressure. The residue thus obtained was purified by preparative chromatography to obtain the title compound. Yield: 202 mg.
$^1$H NMR ($CDCl_3$)δ: 7.22-7.23 (m, 1H), 7.10-7.11 (m, 1H), 6.96-6.98 (m, 1H), 4.24-4.35 (q, 2H), 3.03-3.08 (m, 1H), 1.58-2.19 (m, 6H), 1.30-1.34 (t, 3H).

**Step b: Synthesis of (3*R*)-1-azabicyclo[2.2.2]oct-3-yl (3,3-difluorocyclopentyl)(hydroxy) thiophen-2-ylacetate**

1R,2R + 1S,2S or (1R,2S + 1S,2R)-Ethyl (3,3-difluorocyclopentyl)(hydroxyl)thiophen-2-ylacetate (0.0017 mol, 500 mg) and (*R*)-1-azabicyclo[2.2.2]octan-3-ol (commercially available)(0.0017 mol, 219 mg) were taken in dry benzene (~ 10 ml). To this solution was added sodium metal (0.0008 mol, 20 mg) portionwise. The reaction mixture was heated at about 90 °C for about 4.5 hours. The azeotrope so formed was distilled off continuously. The residue was taken in ethyl acetate and washed with water. The aqueous layer was extracted with ethyl acetate. The combined organic layer was washed with water, brine and dried over anhydrous sodium sulphate and concentrated under reduced pressure. The residue thus obtained was purified by preparative chromatography. Yield: 115mg.
Mass (m/z): 372.14 (M+1).

**Step c: Synthesis of (3*R*)-3-{[(3,3-difluorocyclopentyl)(hydroxy)thiophen-2-ylacetyl]oxy}-1-methyl-1-azoniabicyclo[2.2.2] octane**

To a solution of compound obtained from step b (18 mg) in chloroform (about 0.5 ml) was added methyl bromide (0.5 ml, 25% solution in acetonitrile). The reaction mixture was stirred at 25 °C for about 24 hours. The reaction mixture was triturated with diethyl ether. The precipitates so formed were filtered and washed with diethyl ether and finally dried under high vacuum to give title compound. Yield: 15mg.
Mass (m/z): 386.12 ($M^+$);

The following compounds were prepared by altering the condition of the reaction from room temperature to reflux from one to several days.

(3*R*)-3-{[(3,3-Difluorocyclopentyl)(hydroxy)thiophen-2-ylacetyl]oxy}-1-ethyl-1-azoniabicyclo[2.2.2]octane bromide (Compound No. 15)
Mass (m/z,): 400.26 ($M^+$);

(3*R*)-3-{[(3,3-Difluorocyclopentyl)(hydroxy)thiophen-2-ylacetyl]oxy}-1-propyl-1-azoniabicyclo[2.2.2]octane bromide (Compound No. 16)

Mass (m/z,): 414.30($M^+$);

(3*R*)-1-Butyl-3-{[(3,3-difluorocyclopentyl)(hydroxy)thiophen-2-ylacetyl]oxy}-1-azoniabicyclo[2.2.2]octane bromide (Compound No. 17)
Mass (m/z,): 428.33($M^+$);

(3*R*)-3-{[(3,3-Difluorocyclopentyl)(hydroxy)thiophen-2-ylacetyl]oxy}-1-(2-oxo-2-phenylethyl)-1-azoniabicyclo [2.2.2]octane bromide (Compound No. 18)
Mass (m/z,): 490.23($M^+$);

(3*R*)-3-{[(3,3-Difluorocyclopentyl)(hydroxy)thiophen-2-ylacetyl]oxy}-1-(2-phenylethyl)-1-azoniabicyclo[2.2.2]octane bromide (Compound No. 19)
Mass (m/z,): 476.27($M^+$);

(3*R*)-3-{[(3,3-Difluorocyclopentyl)(hydroxy)thiophen-2-ylacetyl]oxy}-1-[2-(3-fluorophenyl)-2-oxoethyl]-1-azoniabicyclo[2.2.2]octane bromide (Compound No. 20)
Mass (m/z,): 508.50($M^+$).

Example 2: Preparation of (3*R*)-3-{[hydroxy(dithiophen-2-yl)acetyl]oxy}-1-methyl-1-azoniabicyclo[2.2.2]octane bromide (Compound No. 1)

**Step a: Synthesis of (3*R*)-1-azabicyclo[2.2.2]oct-3-yl hydroxy(dithiophen-2-yl)acetate**

To the suspension of (*R*)-1-azabicyclo[2.2.2]octan-3-ol (0.00049 mmol, 63 mg) in dry toluene (about 5 ml) was added freshly prepared sodium ethoxide (0.0006 mol, 43 mg) followed by ethyl hydroxyl (dithiophen-2-yl)acetate (0.0006 mol, 200 mg) under argon atmosphere. The mixture was slowly heated for about 2.5 hours (1 hour at 70 to 80 °C and 1.5 hours at 80 to 95 °C) and the azeotrope formed by the liberated ethanol and the toluene was distilled (~1.5 ml). When the internal temperature reached to 110 to 115°C, ~ 2 ml of distillate was collected. Added about 4 ml of anhydrous toluene and slow distillation was continued. Heating was stopped after - 3 ml of distillate was collected. The reaction was cooled on an ice bath and 2 ml of diethyl ether was added followed by addition of water (2 ml). The two layers so formed were separated. The aqueous layer was extracted with ethyl acetate. The combined organic layers were washed with water and with 1N methane sulphonic acid. The combined aqueous layers were cooled to 0 °C and then basified with concentrated aqueous solution of potassium carbonate. The aqueous layer was extracted with ethyl acetate. The organic layer was washed with water, brine, dried over anhydrous sodium sulphate and concentrated under reduced pressure to furnish the title compound. Yield = 104 mg.
Mass (m/z) (MeOH): 350.19 (M+1)

**Step b: Synthesis of (3*R*)-3-{[hydroxy(dithiophen-2-yl)acetyl]oxy}-1-methyl-1-azoniabicyclo [2.2.2] octane bromide**

To the solution of compound obtained from step *a* (0.00008 mol, 30 mg) above, in chloroform (about 0.5 ml) was added methyl bromide (0.000042 mol, 160 mg, 25% solution in acetonitrile). The reaction mixture was stirred under inert condition at about 25 °C for about 48 hours. The reaction mixture was triturated with diethyl ether. The precipitates so formed were filtered, washed with diethyl ether and finally dried under high vacuum to give title compound. Yield = 22 mg.
Mass (m/z): 364.07 ($M^+$).
Following compounds were prepared similarly;

(3*R*)-1-Benzyl-3-{[hydroxy(dithiophen-2-yl)acetyl]oxy}-1-azoniabicyclo[2.2.2]octane bromide (Compound No. 2)
Mass (m/z): 440.08 ($M^+$);

(3*R*)-1-(4-Bromobenzyl)-3-{[hydroxy(dithiophen-2-yl)acetyl]oxy}-1-azoniabicyclo[2.2.2]octane bromide (Compound No. 3)
Mass (m/z): 519.81 ($M^+$);

(3*R*)-1-Ethyl-3-{[hydroxy(dithiophen-2-yl)acetyl]oxy}-1-azoniabicyclo[2.2.2]octane bromide (Compound No. 4)
Mass (m/z,): 377.80 ($M^+$);

(3*R*)-1-(3-Fluorobenzyl)-3-{[hydroxy(dithiophen-2-yl)acetyl]oxy}-1-azoniabicyclo[2.2.2]octane bromide (Compound No. 5)
Mass (m/z): 458.15 ($M^+$);

(3*R*)-1-(3,5-Difluorobenzyl)-3-{[hydroxy(dithiophen-2-yl)acetyl]oxy}-1-azoniabicyclo[2.2.2]octane bromide (Compound No. 6)
Mass (m/z): 476.21 ($M^+$);

(3*R*)-1-(3-Bromobenzyl)-3-{[hydroxy(dithiophen-2-yl)acetyl]oxy}-1-azoniabicyclo[2.2.2]octane bromide (Compound no. 7)
Mass (m/z): 520.00 ($M^+$);

(3*R*)-1-(4-Fluorobenzyl)-3-{[hydroxy(dithiophen-2-yl)acetyl]oxy}-1-azoniabicyclo[2.2.2]octane bromide (Compound No. 8)
Mass (m/z): 458.12 ($M^+$).

(3*R*)-1-(4-Chlorobenzyl)-3-{[hydroxy(dithiophen-2-yl)acetyl]oxy}-1-azoniabicyclo[2.2.2]octane bromide (Compound No. 9)
Mass (m/z): 474.14 ($M^+$);

(3*R*)-1-(2,5-Difluorobenzyl)-3-{[hydroxy(dithiophen-2-yl)acetyl]oxy}-1-azoniabicyclo[2.2.2]octane bromide (Compound No. 10)
Mass (m/z,): 476.20 ($M^+$);

(3*R*)-3-{[Hydroxy(dithiophen-2-yl)acetyl]oxy}-1-(2-oxo-2-phenylethyl)-1-azoniabicyclo[2.2.2]octane bromide (Compound No. 11)
Mass (m/z,): 468.00 ($M^+$);

(3*R*)-3-{[Hydroxy(dithiophen-2-yl)acetyl]oxy}-1-propyl-1-azomabicyclo[2.2.2]octane bromide (Compound No. 12)
Mass (m/z,): 392.16 ($M^+$);

(3*R*)-1-Butyl-3-{[hydroxy(dithiophen-2-yl)acetyl]oxy}-1-azoniabicyclo[2.2.2]octane bromide (Compound No. 13)
Mass (m/z,): 406.20 ($M^+$).

## Biological Activity

Radioligand Binding Assays:

The affinity of test compounds for $M_1$, $M_2$ and $M_3$ muscarinic receptor subtypes was determined by [$^3$H]-*N*-methylscopolamine binding studies using rat heart and submandibular gland respectively as described by Moriya et al., (Life Sci, 1999, 64(25): 2351-2358) with minor modifications. In competition binding studies, specific binding of [$^3$H] NMS was also determined using membranes from Chinese hamster ovary (CHO) cells expressing cloned human $M_1$, $M_2$, $M_3$, $M_4$ and $M_5$ receptors. Selectivities were calculated from the Ki values obtained on these human cloned membranes.

**Membrane preparation:** Submandibular glands and heart were isolated and placed in ice-cold homogenizing buffer (HEPES 20mM, 10mM EDTA, pH 7.4) immediately after sacrifice. The tissues were homogenized in 10 volumes of homogenizing buffer and the homogenate was filtered through two layers of wet gauze and filtrate was centrifuged at 500g for 10 min. at 4 °C. The supernatant was subsequently centrifuged at 40,000gm for 20 min. at 4 °C. The pellet thus obtained was resuspended in assay buffer (HEPES 20 mM, EDTA 5mM, pH 7.4) and were stored at -70°C until the time of assay.

**Ligand binding assay:** The compounds were dissolved and diluted in DMSO. The membrane homogenates (150-250 μg protein) were incubated in 250 μl of assay volume (HEPES 20 mM, pH 7.4) at 24-25 °C for 3h. Non-specific binding was determined in the presence of 1 μM atropine. The incubation was terminated by vacuum filtration over GF/B fiber filters (Wallac). The filters were then washed with ice-cold 50mM Tris HCl buffer (pH 7.4). The filter mats were dried and bound radioactivity retained on filters was counted. The $IC_{50}$ & $K_d$ were estimated by using the non-linear curve fitting program using G Pad Prism software. The value of inhibition constant $K_i$ was calculated from competitive binding studies by using Cheng & Prusoff equation (Biochem. Pharmacol., 1973, 22: 3099-3108), $Ki = IC_{50} /(1+L/Kd)$, where L is the concentration of [$^3$H]NMS used in the particular experiment. pKi is -log [Ki].

The tested compounds specifically disclosed herein exhibited Ki at $M_3$ receptors in the range of low nanomolar.

For example, the specifically disclosed compounds displayed Ki from about 0.03 nM to about 40 nM, or from about 0.03 nM to about 3.0 nM, or from about 0.03 nM to about 1 nM, or from about 0.03 nM to about 0.3 nM.

The tested compounds specifically disclosed herein exhibited Ki at $M_2$ receptors in the range of low nanomolar. For example, the specifically disclosed compounds displayed Ki from about 0.05 nM to about 20 nM, or from about 0.05 nM to about 1.0 nM or from about 0.05 nM to about 0.4 nM.

The specifically disclosed compounds displayed $M_2/M_3$ Ki in the range of about 0.22 nM to about 2.2 nM, or from about 0.6 nM to about 2.2 nM, or from about 1.0 nM to about 2.2 nM.

**Functional Experiments using isolated rat bladder:**

Methodology:

Animals were euthanized by overdose of thiopentone and whole bladder was isolated and removed rapidly and placed in ice cold Tyrode buffer with the following composition (mMol/L) NaCl 137; KCl 2.7; $CaCl_2$ 1.8; $MgCl_2$ 0.1; $NaHCO_3$ 11.9; $NaH_2PO_4$ 0.4; Glucose 5.55 and continuously gassed with 95% $O_2$ and 5 % $CO_2$.

The bladder was cut into longitudinal strips (3mm wide and 5-6 mm long) and mounted in 10 ml organ baths at 30 °C, with one end connected to the base of the tissue holder and the other end connected through a force displacement transducer. Each tissue was maintained at a constant basal tension of 1 gm and allowed to equilibrate for $1^{1/2}$ hour during which the Tyrode buffer was changed every 15-20 min. At the end of equilibration period the stabilization of the tissue contractile response was assessed with 1μmol/L of carbachol till a reproducible response was obtained. Subsequently a cumulative concentration response curve to carbachol ($10^{-9}$ mol/L to 3 X $10^{-4}$ mol/L) was obtained. After several washes, once the baseline was achieved, cumulative concentration response curve was obtained in presence of NCE (NCE added 20 min. prior to the second cumulative response curve).

The contractile results were expressed as % of control E max. $ED_{50}$ values are calculated by fitting a non-linear regression curve (Graph Pad Prism). pKb values were calculated by the formula pKb = - log [(molar concentration of antagonist/ (dose ratio-1))] where, dose ratio = $ED_{50}$ in the presence of antagonist/$ED_{50}$ in the absence of antagonist.

***In-vitro* functional assay**

Animals and anaesthesia:

The guinea pig (400-600gm) was procured and trachea was removed under anesthesia (sodium pentobarbital, 300 mg/kg i.p) and was immediately kept it in ice-cold Krebs Henseleit (KH) buffer. Indomethacin (10uM) was present throughout the KH buffer to prevent the formation of bronchoactive prostanoids.

Trachea experiments:

The tissue of adherent fascia was cleaned and was cut into strips of equal size (with approx. 4-5 tracheal rings in each strip). The epithelium was removed by careful rubbing, minimizing damage to the smooth muscle. Trachea was opened along the mid-dorsal surface with the smooth muscle band intact and made a series of transverse cuts from alternate sides so that they do not transect the preparation completely. Opposite ends of the cut rings were tied with the help of a thread. The tissue was mounted in isolated tissue baths containing 10ml Krebs Henseleit buffer maintained at 37 °C and bubbled with carbogen, at a basal tension of 1 gm. The buffer was changed 4-5 times for about an hour. The tissue was equilibrated for 1 hr for stabilization. After 1 hr, the tissue was challenged with 1uM carbachol. This was repeated after every 2-3 washes till two similar consecutive responses were obtained. At the end of stabilization, the tissue was washed for 30 minutes followed by incubation with suboptimal dose of MRA/Vehicle for 20 minutes prior to contraction of the tissues with 1μM carbachol. The contractile response of tissues was recorded either on Powerlab data acquisition system or on Grass polygraph (Model 7). The relaxation was expressed as percentage of maximum carbachol response. The data was expressed as mean ± s.e. mean for n observations. The $EC_{50}$ was calculated as the concentration producing 50% of the maximum relaxation to 1μM carbachol. The percent relaxation was compared between the treated and control tissues using non-parametric unpaired t-test. A p value of $< 0.05$ was considered to be statistically significant.

The Compound No. 17 exhibited a pKb of about 9.5.

***In-vitro* functional assay to evaluate efficacy of "MRA" in combination with "PDE-IV inhibitors"**

Animals and anaesthesia:

Trachea tissue is obtained from a guinea pig (400-600gm) under anesthesia (sodium pentobarbital, 300 mg/kg i.p) and is immediately kept in an ice-cold Krebs Henseleit buffer. Indomethacin (10uM) is present throughout the KH buffer to prevent the formation of bronchoactive prostanoids.

Trachea experiments:

Trachea tissue is cleaned off adherent fascia and cut it into strips of equal size (with approx. 4-5 tracheal rings in each strip). The epithelium is removed by careful rubbing, minimizing damage to the smooth muscle. The trachea is opened along the mid-dorsal surface with the smooth muscle band intact and a series of transverse cuts is made from alternate sides so that they do not transect the preparation completely. Opposite ends of the cut rings are tied with the help of a thread. The tissue is mounted in isolated tissue baths containing 10 mL Krebs Henseleit buffer maintained at 37°C and is bubbled with carbogen, at a basal tension of 1 gm. The buffer is changed 4-5 times for about an hour and the tissue is equilibrated for 1 hour for stabilization. After 1 hour, the tissue is contacted with 1uM carbachol. Repeat this after every 2-3 washes until two similar consecutive responses are obtained. At the end of stabilization, the tissue is washed for 30 minutes followed by incubation with suboptimal dose of MRA/Vehicle for 20 minutes prior to contraction of the tissues with 1μM carbachol. The relaxant activity of the PDE-IV inhibitor [$10^{-9}$ M to $10^{-4}$ M] on the stabilized developed tension/response is assessed. The contractile response of tissues is recorded either on a Powerlab data acquisition system or on a Grass polygraph (Model 7). The relaxation is expressed as a percentage of maximum carbachol response. The data is expressed as mean ± s.e. mean for n observations. The $EC_{50}$ is calculated as the concentration producing 50% of the maximum relaxation to 1μM carbachol. The percent relaxation between the treated and control tissues are compared using non-parametric unpaired t-test. A p value of $< 0.05$ is considered to be statistically significant.

***In-vivo* assay to evaluate efficacy of MRA inhibitors**

Male Guinea pigs were anesthetized with urethane (1.5 g/kg, i.p.). Trachea was cannulated along with jugular vein (for carbachol challenge) and animals were placed in the Plethysmograph-Box (PLY 3114 model; Buxco Electronics, Sharon, USA.). Respiratory parameters were recorded using Pulmonary Mechanics Analyzer, Biosystems XA software (Buxco Electronics, USA), which calculated lung resistance ($R_L$) on a breath-by-breath basis. Bronchoconstriction was induced by injections of Carbachol (10 μg/kg) delivered into the jugular vein. Increase in $R_L$ over a period of 5 min post carbachol challenge was recorded in presence or absence of MRA or vehicle at 2 hrs and 12 hrs post treatment and expressed as % increase in $R_L$ from basal

***In-vivo* assay to evaluate efficacy of MRA in combination with PDE-IV inhibitors**

Drug treatment:

MRA (1μg/kg to 1mg/kg) and PDE-IV inhibitor (1μg/kg to 1mg/kg) are instilled intratracheally under anesthesia either alone or in combination.

Method:

Male wistar rats weighing 200±20gm are used in the study. Rats have free access to food and water. On the day of experiment, animals are exposed to lipopolysaccharide (LPS, 100μg/ml) for 40 min. One group of vehicle treated rats is exposed to phosphate buffered saline (PBS) for 40 min. Two hours after LPS/PBS exposure, animals are placed inside a whole body plethysmograph (Buxco Electronics, USA) and exposed to PBS or increasing acetylcholine (1, 6, 12, 24, 48 and 96 mg/ml) aerosol until Penh values (index of airway resistance) of rats attained 2 times the value (PC-100) seen with PBS alone. The respiratory parameters are recorded online using Biosystem XA software, (Buxco Electronics, USA). Penh, at any chosen dose of acetylcholine is, expressed as percent of PBS response and the using a nonlinear regression analysis PC100 (2 folds of PBS value) values are computed. Percent inhibition is computed using the following formula.

$$\% \text{ Inhibition} = \frac{PC100_{LPS} - PC100_{TEST}}{PC100_{LPS} - PC100_{PBS}} \times 100$$

Where,
$PC100_{LPS}$ = PC100 in untreated LPS challenged group
$PC100_{TEST}$ = PC100 in group treated with a given dose of test compound
$PC100_{PBS}$ = PC100 in group challenged with PBS

Immediately after the airway hyper reactivity response is recorded, animals are sacrificed and bronchoalveolar lavage (BAL) is performed. Collected lavage fluid is centrifuged at 3000 rpm for 5 min, at 4°C. Total leukocyte count is performed in the resuspended sample. A portion of suspension is cytocentrifuged and stained with Leishmann's stain for differential leukocyte count. Total leukocyte and Neutrophil counts are expressed as cell count (millions cells $ml^{-1}$ of BAL). Percent inhibition is computed using the following formula.

$$\% \text{ Inhibition} = \frac{NC_{LPS} - NC_{TEST}}{NC_{LPS} - NC_{CON}} \times 100$$

Where,
$NC_{LPS}$ = Percentage of neutrophil in untreated LPS challenged group
$NC_{TEST}$ =Percentage of neutrophil in group treated with a given dose of test compound
$NC_{CON}$ = Percentage of neutrophil in group not challenged with LPS
The percent inhibition data is used to compute $ED_{50}$ vales using Graph Pad Prism software (Graphpad Software Inc., USA).

**<u>*In-vivo* assay to evaluate efficacy of MRA in combination with Corticosteroids</u>**

<u>Ovalbumin induced airway inflammation:</u>

Guinea pigs are sensitized on days 0, 7 and 14 with 50-μg ovalbumin and 10 mg aluminum hydroxide injected intraperitoneally. On days 19 and 20 guinea pigs are exposed to 0.1% w $v^{-1}$ ovalbumin or PBS for 10 min, and with 1% ovalbumin for 30 min on day 21. Guinea pigs are treated with test compound (0.1, 0.3 and 1 mg $kg^{-1}$) or standard 1 mg $kg^{-1}$ or vehicle once daily from day 19 and continued for 4 days. Ovalbumin/PBS challenge is performed 2 hours after different drug treatment.

Twenty four hours after the final ovalbumin challenge BAL is performed using Hank's balanced salt solution (HBSS). Collected lavage fluid is centrifuged at 3000 rpm for 5 min, at 4°C. Pellet is collected and resuspended in 1ml HBSS. Total leukocyte count is performed in the resuspended sample. A portion of suspension is cytocentrifuged and stained with Leishmann's stain for differential leukocyte count. Total leukocyte and eosinophil count are expressed as cell count (millions cells $ml^{-1}$ of BAL). Eosinophil is also expressed as percent of total leukocyte count. % inhibition is computed using the following formula.

$$\% \text{ Inhibition} = \frac{Eos_{OVA} - Eos_{TEST}}{Eos_{OVA} - Eos_{CON}} \times 100$$

Where,
$Eos_{OVA}$ = Percentage of eosinophil in untreated ovalbumin challenged group
$Eos_{TEST}$ =Percentage of eosinophil in group treated with a given dose of test compound
$Eos_{CON}$ = Percentage of eosinophil in group not challenged with ovalbumin.

***In-vivo* assay to evaluate efficacy of "MRA" in combination with p38 MAP Kinase inhibitors**

Lipopolysaccharide (LPS) induced airway hyper reactivity (AHR) and neutrophilia:

Drug treatment:
MRA (1μg/kg to 1mg/kg) and p38 MAP kinase inhibitor (1μg/kg to 1mg/kg) are instilled intratracheally under anesthesia either alone or in combination.
Method:
Male wistar rats weighing 200±20gm are used in the study. Rats have free access to food and water. On the day of experiment, animals are exposed to lipopolysaccharide (LPS, 100μg/ml) for 40 min. One group of vehicle treated rats is exposed to phosphate buffered saline (PBS) for 40 min. Two hours after LPS/PBS exposure, animals are placed inside a whole body plethysmograph (Buxco Electronics, USA) and exposed to PBS or increasing acetylcholine (1, 6, 12, 24, 48 and 96 mg/ml) aerosol until Penh values (index of airway resistance) of rats attained 2 times the value (PC-100) seen with PBS alone. The respiratory parameters are recorded online using Biosystem XA software, (Buxco Electronics, USA). Penh, at any chosen dose of acetylcholine is, expressed as percent of PBS response and the using a nonlinear regression analysis PC100 (2 folds of PBS value) values are computed. Percent inhibition is computed using the following formula.

$$\% \text{ Inhibition} = \frac{PC100_{LPS} - PC100_{TEST}}{PC100_{LPS} - PC100_{PBS}} \times 100$$

Where,
$PC100_{LPS}$ = PC100 in untreated LPS challenged group
$PC100_{TEST}$ = PC100 in group treated with a given dose of test compound
$PC100_{PBS}$ = PC100 in group challenged with PBS

Immediately after the airway hyper reactivity response is recorded, animals are sacrificed and bronchoalveolar lavage (BAL) is performed. Collected lavage fluid is centrifuged at 3000 rpm for 5 min, at 4°C. Pellet is collected and resuspended in 1ml HBSS. Total leukocyte count is performed in the resuspended sample. A portion of suspension is cytocentrifuged and stained with Leishmann's stain for differential leukocyte count. Total leukocyte and Neutrophil counts are expressed as cell count (millions cells $ml^{-1}$ of BAL). Percent inhibition is computed using the following formula.

$$\% \text{ Inhibition} = \frac{NC_{LPS} - NC_{TEST}}{NC_{LPS} - NC_{CON}} \times 100$$

Where,
$NC_{LPS}$ = Percentage of neutrophil in untreated LPS challenged group
$NC_{TEST}$ =Percentage of neutrophil in group treated with a given dose of test compound
$NC_{CON}$ = Percentage of neutrophil in group not challenged with LPS
The percent inhibition data is used to compute $ED_{50}$ values using Graph Pad Prism software (Graphpad Software Inc.,USA).

***In-vivo* assay to evaluate efficacy of "MRA" in combination with β2-agonists**

Drug treatment:

MRA (1μg/kg to 1mg/kg) and long acting $\beta_2$ agonist is instilled intratracheally under anesthesia either alone or in combination.

Method

Wistar rats (250-350gm) or balb/C mice (20-30gm) is placed in body box of a whole body plethysmograph (Buxco Electronics., USA) to induce bronchoconstriction. Animals are allowed to acclimatize in the body box and are given successive challenges, each of 2 min duration, with PBS (vehicle for acetylcholine) or acetylcholine (i.e. 24, 48, 96, 144, 384, and 768 mg/ml). The respiratory parameters are recorded online using Biosystem XA software, (Buxco Electronics, USA) for 3 min. A gap of 2 min is allowed for the animals to recover and then challenged with the next higher dose of acetylcholine (ACh). This step is repeated until Penh of rats attained 2 times the value (PC-100) seen with PBS challenge. Following PBS/ACh challenge, Penh values (index of airway resistance) in each rat/mice is obtained in the presence of PBS and different doses of ACh. Penh, at any chosen dose of ACh is, expressed as percent of PBS response. The Penh values thus calculated are fed into Graph Pad Prism software (Graphpad Software Inc., USA) and using a nonlinear regression analysis PC100 (2 folds of PBS value) values are computed. % inhibition is computed using the following formula.

$$\%\ \text{Inhibition} = \frac{PC100_{TEST} - PC100_{CON}}{768 - PC100_{CON}} \times 100$$

Where,
$PC100_{CON}$ = PC100 in vehicle treated group
$PC100_{TEST}$ = PC100 in group treated with a given dose of test compound
768 = is the maximum amount of acetylcholine used.

**Claims**

**1.** A compound of Formula I:

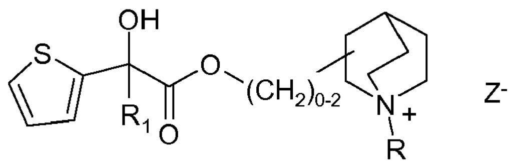

**Formula I**

and their pharmaceutically acceptable solvates, enantiomers, diastereomers, polymorphs or *N*-oxides
wherein
$R_1$ is thienyl, or cycloalkyl substituted with difluoro on same carbon;
R is $C_{1-4}$ linear alkyl;
or
R is alkyl which is substituted with phenyl or phenyl carbonyl where phenyl is optionally substituted with one or more halogen atoms, hydroxyl, alkyl, alkenyl, alkynyl, cycloalkyl, alkoxy, acyl, aryloxy, cyano, nitro, $-COOR_c$, $-NHC(=O)R_b$, $-NR_bR_d$, $-C(=O)NR_bR_d$, $-NHC(=O)NR_bR_d$, $-OC(=O)NR_bR_d$, $-SO_mR_c$, heterocyclyl, heteroaryl, heterocyclylalkyl, heteroarylalkyl, $-SR_b$, haloalkyl;
$R_b$ and $R_d$ are independently selected from hydrogen, alkyl, alkenyl, alkynyl, alkoxy, cycloalkyl, aryl, aralkyl, heterocyclyl, heteroaryl, heterocyclylalkyl, heteroarylalkyl, or carboxy;
$R_c$ is hydrogen, alkyl, alkenyl, alkynyl, cycloalkyl, aralkyl, aryl, heterocyclyl, heteroaryl, heteroarylalkyl or heterocyclylalkyl;
m is an integer from 0 to 2;
Z is an anion selected from acetate, succinate, maleate, methanesulphonate, benzenesulphonate, trifluoroacetate, oxalate, tartarate, citrate, glutamate, fumarate, malonate, adepate, ascorbate, carbonate, camphoenate acid, nicotinate, butyrate, tartarate, lactate, sulphate, phosphate, glucurinate, chloride, bromide, hexafluorophosphate, nitrate,

borate or perchlorate.

2. A compound selected from the group consisting of :

(3*R*)-3-{[Hydroxyl(dithiophen-2-yl)acetyl]oxy}-1-methyl-1-azoniabicyclo[2.2.2]octane bromide (Compound No. 1);
(3*R*)-1-Benzyl-3-{[hydroxyl(dithiophen-2-yl)acetyl]oxy}-1-azoniabicyclo[2.2.2]octane bromide (Compound No. 2);
(3*R*)-1-(4-Bromobenzyl)-3-{[hydroxy(dithiophen-2-yl)acetyl]oxy}-1-azoniabicyclo[2.2.2]octane bromide (Compound No. 3);
(3*R*)-1-Ethyl-3-{[hydroxy(dithiophen-2-yl)acetyl]oxy}-1-azoniabicyclo[2.2.2]octane bromide (Compound No. 4);
(3*R*)-1-(3-Fluorobenzyl)-3-{[hydroxy(dithiophen-2-yl)acetyl]oxy}-1-azoniabicyclo[2.2.2]octane bromide (Compound No. 5);
(3*R*)-1-(3,5-Difluorobenzyl)-3-{[hydroxy(dithiophen-2-yl)acetyl]oxy}-1-azoniabicyclo[2.2.2]octane bromide (Compound No. 6);
(3*R*)-1-(3-Bromobenzyl)-3-{[hydroxy(dithiophen-2-yl)acetyl]oxy}-1-azoniabicyclo[2.2.2]octane bromide (Compound No. 7);
(3*R*)-1-(4-Fluorobenzyl)-3-{[hydroxy(dithiophen-2-yl)acetyl]oxy}-1-azoniabicyclo[2.2.2]octane bromide (Compound No. 8);
(3*R*)-1-(4-Chlorobenzyl)-3-{[hydroxy(dithiophen-2-yl)acetyl]oxy}-1-azoniabicyclo[2.2.2]octane bromide (Compound No. 9);
(3*R*)-1-(2,5-Difluorobenzyl)-3-{[hydroxy(dithiophen-2-yl)acetyl]oxy}-1-azoniabicyclo[2.2.2]octane bromide (Compound No. 10);
(3*R*)-3-{[Hydroxy(dithiophen-2-yl)acetyl]oxy}-1-(2-oxo-2-phenylethyl)-1-azoniabicyclo [2.2.2] octane bromide (Compound No. 11);
(3*R*)-3-{[Hydroxy (dithiophen-2-yl) acetyl] oxy}-1-propyl-1-azoniabicyclo[2.2.2] octane bromide (Compound No. 12);
(3*R*)-1-Butyl-3-{[hydroxy(dithiophen-2-yl) acetyl] oxy}-1-azoniabicyclo [2.2.2] octane bromide (Compound No. 13);
(3*R*)-3-{[(3,3-Difluorocyclopentyl)(hydroxy)thiophen-2-ylacetyl]oxy}-1-methyl-1-azoniabicyclo[2.2.2]octane bromide (Compound No. 14);
(3*R*)-3-{[(3,3-Difluorocyclopentyl)(hydroxy)thiophen-2-ylacetyl]oxy}-1-ethyl-1-azoniabicyclo[2.2.2]octane bromide (Compound No. 15);
(3*R*)-3-{[(3,3-Difluorocyclopentyl)(hydroxy)thiophen-2-ylacetyl]oxy}-1-propyl-1-azoniabicyclo[2.2.2]octane bromide (Compound No. 16);
(3*R*)-1-Butyl-3-{[(3,3-difluorocyclopentyl)(hydroxy)thiophen-2-ylacetyl]oxy}-1-azoniabicyclo[2.2.2]octane bromide (Compound No. 17);
(3*R*)-3-{[(3,3-Difluorocyclopentyl)(hydroxy)thiophen-2-ylacetyl]oxy}-1-(2-oxo-2-phenylethyl)-1-azoniabicyclo [2.2.2]octane bromide (Compound No. 18);
(3*R*)-3-{[(3,3-Difluorocyclopentyl)(hydroxy)thiophen-2-ylacetyl]oxy}-1-(2-phenylethyl)-1-azoniabicyclo[2.2.2] octane bromide (Compound No. 19);
(3*R*)-3-{[(3,3-Difluorocyclopentyl)(hydroxy)thiophen-2-ylacetyl]oxy}-1-[2-(3-fluorophenyl)-2-oxoethyl]-1-azoniabicyclo [2.2.2] octane bromide (Compound No. 20).

3. A pharmaceutical composition comprising a therapeutically effective amount of compounds as defined in claim 1 and 2 together with pharmaceutically acceptable carriers, excipient or diluents.

4. A compound according to claim 1 or 2 for us in treating or preventing disease or disorder of the respiratory, urinary and gastrointestinal systems, wherein the disease or disorder is mediated through muscarinic receptors in mammal.

5. The compound of claim 4 for use in treating or preventing urinary incontinence, lower urinary tract symptoms (LUTS), bronchial asthma, chronic obstructive pulmonary disorders (COPD), pulmonary fibrosis, irritable bowel syndrome, obesity, diabetes or gastrointestinal hyperkinesis in mammal.

6. The use of compounds according to claim 1 and 2 for the manufacture of medicament for treating or preventing disease or disorder of the respiratory, urinary and gastrointestinal systems, wherein the disease or disorder is mediated through muscarinic receptors in mammal.

**7.** The use of compounds according to claim 6 for the manufacture of medicament for treating or preventing urinary incontinence, lower urinary tract symptoms (LUTS), bronchial asthma, chronic obstructive pulmonary disorders (COPD), pulmonary fibrosis, irritable bowel syndrome, obesity, diabetes or gastrointestinal hyperkinesis in mammal.

**8.** A pharmaceutical composition according to claim 3 for use in treating or preventing disease or disorder of the respiratory, urinary and gastrointestinal systems, wherein the disease or disorder is mediated through muscarinic receptors in mammal.

**9.** The pharmaceutical composition of claim 8, for use in treating or preventing urinary incontinence, lower urinary tract symptoms (LUTS), bronchial asthma, chronic obstructive pulmonary disorders (COPD), pulmonary fibrosis, irritable bowel syndrome, obesity, diabetes or gastrointestinal hyperkinesis in mammal.

**10.** The use of pharmaceutical composition according to claim 3 for the manufacture of medicament for treating or preventing disease or disorder of the respiratory, urinary and gastrointestinal systems, wherein the disease or disorder is mediated through muscarinic receptors in mammal.

**11.** The use of pharmaceutical composition according to claim 10 for the manufacture of medicament for treating or preventing urinary incontinence, lower urinary tract symptoms (LUTS), bronchial asthma, chronic obstructive pulmonary disorders (COPD), pulmonary fibrosis, irritable bowel syndrome, obesity, diabetes or gastrointestinal hyperkinesis in mammal.

**12.** A pharmaceutical composition comprising one or more compounds according to claim 1 and 2 and which further comprises one or more therapeutic agent selected from one or more corticosteroids, beta agonists, leukotriene antagonists, 5-lipoxygenase inhibitors, anti-histamines, antitussives, dopamine receptor antagonists, chemokine inhibitors, p38 MAP Kinase inhibitors and PDE-IV inhibitors

**13.** A method of preparing a compound of Formula I and its pharmaceutically acceptable solvates, esters, enantiomers, diastereomers, *N*-oxides, or polymorphs, wherein the reaction comprises:

a. reacting a compound of Formula II

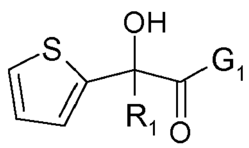

Formula II

with a compound of Formula III

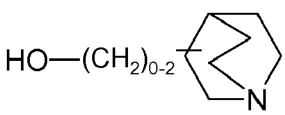

Formula III

to give a compound of Formula IV

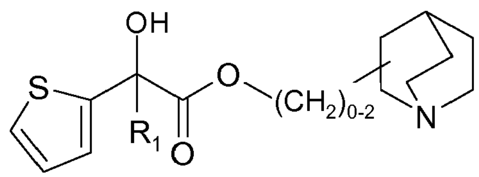

Formula IV

b. compound of Formula IV is *N*-derivatized with a compound of Formula V

R-Z **Formula V**

to give a compound of Formula I

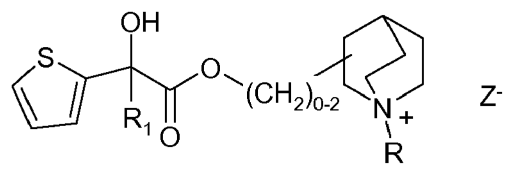

**Formula I**

wherein

$R_1$ is thienyl, or cycloalkyl substituted with difluoro on same carbon;

$G_1$ is hydroxyl or -Oalkyl;

R is $C_{1-4}$ linear alkyl or R is alkyl which is substituted with phenyl or phenyl carbonyl where phenyl is optionally substituted with one or more halogen, hydroxyl, alkyl, alkenyl, alkynyl, cycloalkyl, alkoxy, acyl, aryloxy, cyano, nitro, $-COOR_c$, $-NHC(=O)R_b$, $-NR_bR_d$, $-C(=O)NR_bR_d$, $-NHC(=O)NR_bR_d$, $-OC(=O)NR_bR_d$, $-SO_mR_c$, heterocyclyl, heteroaryl, heterocyclylalkyl, heteroarylalkyl, $-SR_b$, haloalkyl; wherein

$R_b$ and $R_d$ are independently selected from hydrogen, halogen, hydroxyl, alkyl, alkenyl, alkynyl, alkoxy, cycloalkyl, aryl, aralkyl, heterocyclyl, heteroaryl, heterocyclylalkyl, heteroarylalkyl, or carboxy;

$R_c$ is hydrogen, alkyl, alkenyl, alkynyl, cycloalkyl, aralkyl, aryl, heterocyclyl, heteroaryl, heteroarylalkyl or heterocyclylalkyl;

m is an integer from 0 to 2;

Z is an anion selected from acetate, succinate, maleate, methanesulphonate, benzenesulphonate, trifluoroacetate, oxalate, tartarate, citrate, glutamate, fumarate, malonate, adepate, ascorbate, carbonate, camphoenate acid, nicotinate, butyrate, tartarate, lactate, sulphate, phosphate, glucurinate, chloride, bromide, hexafluorophosphate, nitrate, borate or perchlorate.

Europäisches Patentamt
European Patent Office
Office européen des brevets

# EUROPEAN SEARCH REPORT

Application Number
EP 09 16 1744

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2004/096800 A (NOVARTIS AG [CH]; NOVARTIS PHARMA GMBH [AT]; COLLINGWOOD STEPHEN PAUL) 11 November 2004 (2004-11-11) * examples 70-79,113,114,159,166,168,170,246,247 * * claim 11; compounds RN 787628-97-1, RN 787628-85-7, RN 787628-84-6 * * claim 10; compounds RN 787628-94-8 * * claims 1,15-17 * * page 21, line 1 - line 10 * * page 22, lines 7,22 * ----- | 1,3-12 | INV. C07D453/02 A61K31/439 A61P11/00 A61P11/06 A61P13/00 A61P1/00 A61P3/04 |
| X | WO 01/04118 A (ALMIRALL PRODESFARMA SA [ES]; FERNANDEZ FORNER DOLORS [ES]; PRAT QUINO) 18 January 2001 (2001-01-18) * examples 36-87 * * claims 1,22,31,33 * * page 27, line 10 - line 19 * schemes on pages 11 and 12 ----- | 1,3-11, 13 | |
| X | WO 2005/090342 A (ALMIRALL PRODESFARMA SA [ES]; FERNANDEZ FORNER MARIA DOLORS [ES]; PRAT) 29 September 2005 (2005-09-29) * examples 5-30 * * claims 1,18,19,21,22 * * page 30, line 9 - line 16 * * page 30, line 35 - page 31, line 3 * scheme on page 18 ----- | 1-13 | TECHNICAL FIELDS SEARCHED (IPC): C07D |
| X | WO 2006/048225 A (NOVARTIS AG [CH]; NOVARTIS PHARMA GMBH [AT]; PRESS NEIL JOHN [GB]; COL) 11 May 2006 (2006-05-11) * examples 40,72,94,125,152,179,204,217 * * claims 1,8,10 * * page 9, line 28 * * page 10, line 5 - line 17 * ----- | 1,3-12 | |

The present search report has been drawn up for all claims

| Place of search | Date of completion of the search | Examiner |
|---|---|---|
| Munich | 30 July 2009 | Brandstetter, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

## ANNEX TO THE EUROPEAN SEARCH REPORT ON EUROPEAN PATENT APPLICATION NO. EP 09 16 1744

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2009

| Patent document cited in search report | Publication date | Patent family member(s) | Publication date |
|---|---|---|---|
| WO 2004096800 A | 11-11-2004 | AU 2004234069 A1 | 11-11-2004 |
| | | BR PI0410238 A | 16-05-2006 |
| | | CA 2523436 A1 | 11-11-2004 |
| | | CL 9182004 A1 | 11-02-2005 |
| | | EC SP056134 A | 19-04-2006 |
| | | EP 1631569 A2 | 08-03-2006 |
| | | HR 20050933 A2 | 31-12-2006 |
| | | IS 8158 A | 30-11-2005 |
| | | JP 2006525267 T | 09-11-2006 |
| | | KR 20060015572 A | 17-02-2006 |
| | | MA 27775 A1 | 01-02-2006 |
| | | MX PA05011739 A | 26-01-2006 |
| | | NZ 542995 A | 28-02-2009 |
| | | US 2007060563 A1 | 15-03-2007 |
| WO 0104118 A | 18-01-2001 | AR 029760 A1 | 16-07-2003 |
| | | AT 235492 T | 15-04-2003 |
| | | AU 779881 B2 | 17-02-2005 |
| | | AU 6433000 A | 30-01-2001 |
| | | AU 2005202144 A1 | 09-06-2005 |
| | | BG 65565 B1 | 30-12-2008 |
| | | BG 106301 A | 30-08-2002 |
| | | BR 0012434 A | 02-04-2002 |
| | | CA 2381165 A1 | 18-01-2001 |
| | | CN 1373760 A | 09-10-2002 |
| | | CN 1824664 A | 30-08-2006 |
| | | CZ 20020121 A3 | 13-11-2002 |
| | | DE 60001840 D1 | 30-04-2003 |
| | | DE 60001840 T2 | 11-12-2003 |
| | | DK 1200431 T3 | 21-07-2003 |
| | | EE 200200017 A | 15-04-2003 |
| | | EG 24066 A | 08-05-2008 |
| | | EP 1200431 A2 | 02-05-2002 |
| | | ES 2165768 A1 | 16-03-2002 |
| | | ES 2193098 T3 | 01-11-2003 |
| | | HK 1042487 A1 | 18-07-2003 |
| | | HU 0202100 A2 | 28-10-2002 |
| | | JP 4030040 B2 | 09-01-2008 |
| | | JP 2003504368 T | 04-02-2003 |
| | | JP 2005350476 A | 22-12-2005 |
| | | KR 20070009744 A | 18-01-2007 |
| | | KR 20070007396 A | 15-01-2007 |
| | | NO 20020180 A | 13-03-2002 |
| | | PL 357160 A1 | 12-07-2004 |
| | | PT 1200431 E | 31-07-2003 |
| | | RU 2306312 C2 | 20-09-2007 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 09 16 1744

This annex lists the patent family members relating to the patent documents cited in the above-mentioned European search report.
The members are as contained in the European Patent Office EDP file on
The European Patent Office is in no way liable for these particulars which are merely given for the purpose of information.

30-07-2009

| Patent document cited in search report | Publication date | Patent family member(s) | Publication date |
|---|---|---|---|
| WO 0104118 A | | SI 1200431 T1 | 31-10-2003 |
| | | SK 432002 A3 | 01-04-2003 |
| | | TR 200200768 T2 | 22-07-2002 |
| | | TW 284644 B | 01-08-2007 |
| | | UA 73509 C2 | 15-04-2002 |
| | | US 2003055080 A1 | 20-03-2003 |
| | | US 2008221155 A1 | 11-09-2008 |
| | | US 2005209272 A1 | 22-09-2005 |
| | | US 2007099953 A1 | 03-05-2007 |
| | | US 2004132768 A1 | 08-07-2004 |
| | | UY 26244 A1 | 29-12-2000 |
| | | ZA 200200232 A | 10-04-2003 |
| WO 2005090342 A | 29-09-2005 | AR 048004 A1 | 15-03-2006 |
| | | AT 399166 T | 15-07-2008 |
| | | AU 2005223310 A1 | 29-09-2005 |
| | | BR PI0508177 A | 07-08-2007 |
| | | CA 2560157 A1 | 29-09-2005 |
| | | CN 1930158 A | 14-03-2007 |
| | | DK 1725552 T3 | 13-10-2008 |
| | | EC SP066770 A | 16-11-2006 |
| | | EP 1725552 A1 | 29-11-2006 |
| | | ES 2307168 T3 | 16-11-2008 |
| | | ES 2239546 A1 | 16-09-2005 |
| | | HK 1101855 A1 | 30-04-2009 |
| | | HR 20080338 T3 | 31-07-2008 |
| | | JP 2007529444 T | 25-10-2007 |
| | | KR 20070003940 A | 05-01-2007 |
| | | SI 1725552 T1 | 31-10-2008 |
| | | US 2008214600 A1 | 04-09-2008 |
| | | ZA 200605931 A | 28-11-2007 |
| WO 2006048225 A | 11-05-2006 | AR 054090 A1 | 06-06-2007 |
| | | AU 2005300710 A1 | 11-05-2006 |
| | | BR PI0517945 A | 21-10-2008 |
| | | CA 2583237 A1 | 11-05-2006 |
| | | CN 101056634 A | 17-10-2007 |
| | | EC SP077424 A | 30-05-2007 |
| | | EP 1811999 A1 | 01-08-2007 |
| | | JP 2008518890 T | 05-06-2008 |
| | | KR 20070073849 A | 10-07-2007 |
| | | US 2009048281 A1 | 19-02-2009 |
| | | ZA 200702772 A | 27-08-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004005252 A
- WO 2004004629 A
- WO 2004052857 A
- WO 2004067510 A
- WO 2004014853 A
- WO 2004014363 A
- WO 2004056811 A
- WO 2004056810 A
- WO 2004056767 A
- WO 9914200 A
- WO 03027060 A
- US 6200991 B
- WO 0056718 A
- WO 2004089363 A
- WO 2004089898 A
- WO 2004069835 A
- WO 2004089900 A
- WO 2004089364 A
- WO 2006018708 A
- WO 2006035303 A
- WO 0104118 A
- WO 2004096800 A
- WO 2005090342 A


### Non-patent literature cited in the description


- *Nature,* 1986, vol. 323, 411
- *Science,* 1987, vol. 237, 527
- *Curr. Opin. Chem. Biol,* 1999, vol. 3, 426
- **Eglen.** *Trends in Pharmacol. Sci.,* 2001, vol. 22, 409
- *Molecules,* 2001, vol. 6, 142
- **Birdsall et al.** *Trends in Pharmacol. Sci.,* 2001, vol. 22, 215
- **T.W. Greene ; P.G.M. Wuts.** Protective Groups in Organic Synthesis. John Wiley and Sons
- *J. Am. Chem. Soc.,* 1951, vol. 73, 2216-2218
- **Moriya et al.** *Life Sci,* 1999, vol. 64 (25), 2351-2358
- **Cheng ; Prusoff.** *Biochem. Pharmacol.,* 1973, vol. 22, 3099-3108